# EUROPEAN PATENT SPECIFICATION

(11) **EP 2 194 941 B1**
(45) Date of publication and mention of the grant of the patent: **10.07.2013**
(21) Application number: 08833245.7
(22) Date of filing: 23.09.2008
(51) Int. Cl.: A61F 9/007

(54) **Ocular implants**
Augenimplantate
Implants oculaires

(30) Priority: 24.09.2007 US 860318; 04.03.2008 US 33746
(43) Date of publication of application: 16.06.2010
(60) Divisional application: 13169532.2
(73) Proprietor: Ivantis, Inc., Irvine, CA 92618 (US)
(72) Inventor: SCHIEBER, Andrew, T., Irvine, CA 92618 (US); EUTENEUER, Charles, L., St. Michael, MN 55376 (US)
(74) Representative: Price, Nigel John King
(86) International application number: PCT/US2008/077380
(87) International publication number: WO 2009/042596

(56) References cited:
- DE-A1- 19 840 047
- US-A1- 2002 013 546
- US-A1- 2004 260 228
- US-A1- 2006 195 187

## Description

### FIELD OF THE INVENTION

The present invention relates generally to devices that are implanted within the eye. More particularly, the present invention relates to devices that facilitate the transfer of fluid from within one area of the eye to another area of the eye.

### BACKGROUND OF THE INVENTION

According to a draft report by The National Eye Institute (NEI) at The United States National Institutes of Health (NIH), glaucoma is now the leading cause of irreversible blindness worldwide and the second leading cause of blindness, behind cataract, in the world. Thus, the NEI draft report concludes, "it is critical that significant emphasis and resources continue to be devoted to determining the pathophysiology and management of this disease." Glaucoma researchers have found a strong correlation between high intraocular pressure and glaucoma. For this reason, eye care professionals routinely screen patients for glaucoma by measuring intraocular pressure using a device known as a tonometer. Many modem tonometers make this measurement by blowing a sudden puff of air against the outer surface of the eye.

The eye can be conceptualized as a ball filled with fluid. There are two types of fluid inside the eye. The cavity behind the lens is filled with a viscous fluid known as vitreous humor. The cavities in front of the lens are filled with a fluid know as aqueous humor. Whenever a person views an object, he or she is viewing that object through both the vitreous humor and the aqueous humor.

Whenever a person views an object, he or she is also viewing that object through the cornea and the lens of the eye. In order to be transparent, the cornea and the lens can include no blood vessels. Accordingly, no blood flows through the cornea and the lens to provide nutrition to these tissues and to remove wastes from these tissues. Instead, these functions are performed by the aqueous humor. A continuous flow of aqueous humor through the eye provides nutrition to portions of the eye (e.g., the cornea and the lens) that have no blood vessels. This flow of aqueous humor also removes waste from these tissues.

Aqueous humor is produced by an organ known as the ciliary body. The ciliary body includes epithelial cells that continuously secrete aqueous humor. In a healthy eye, a stream of aqueous humor flows out of the anterior chamber of the eye through the trabecular meshwork and into Schlemm's canal as new aqueous humor is secreted by the epithelial cells of the ciliary body. This excess aqueous humor enters the venous blood stream from Schlemm's canal and is carried along with the venous blood leaving the eye.

When the natural drainage mechanisms of the eye stop functioning properly, the pressure inside the eye begins to rise. Researchers have theorized prolonged exposure to high intraocular pressure causes damage to the optic nerve that transmits sensory information from the eye to the brain. This damage to the optic nerve results in loss of peripheral vision. As glaucoma progresses, more and more of the visual field is lost until the patient is completely blind.

In addition to drug treatments, a variety of surgical treatments for glaucoma have been performed. For example, shunts were implanted to direct aqueous humor from the anterior chamber to the extraocular vein (Lee and Scheppens, "Aqueous-venous shunt and intraocular pressure," Investigative Ophthalmology (Feb. 1966)). Other early glaucoma treatment implants led from the anterior chamber to a sub-conjunctival bleb (e.g., US 4,968,296 and US 5,180,362). Still others were shunts leading from the anterior chamber to a point just inside Schlemm's canal (Spiegel et al., "Schlemm's canal implant: a new method to lower intraocular pressure in patients with POAG?" Ophthalmic Surgery and Lasers (June 1999); US 6,450,984; US 6,450,984). In addition to drug treatments, a variety of surgical treatments for glaucoma have been performed. For example, shunts were implanted to direct aqueous humor from the anterior chamber to the extraocular vein (Lee and Scheppens, "Aqueous-venous shunt and intraocular pressure," Investigative Ophthalmology (Feb. 1966)). Other early glaucoma treatment implants led from the anterior chamber to a sub-conjunctival bleb (e.g., US 4,968,296 and US 5,180,362). Still others were shunts leading from the anterior chamber to a point just inside Schlemm's canal (Spiegel et al., "Schlemm's canal implant: a new method to lower intraocular pressure in patients with POAG?" Ophthalmic Surgery and Lasers (June 1999); US 6,450,984; US 6,450,984).

### SUMMARY OF THE INVENTION

While some prior glaucoma treatment implants did provide a flow path between the anterior chamber and Schlemm's canal, these prior devices failed to recognize (1) the importance of supporting a significant portion of Schlemm's canal in a patent state or (2) the harm to adjacent tissue caused by relatively high fluid flow rates at or around any portion of the device. The ocular implant devices of this invention address one or both of these design criteria.

The ocular implant according to the present invention is defined in claim 1. The ocular implant may be inserted into Schlemm's canal of an eye to facilitate the flow of aqueous humor out of the anterior chamber of the eye by, e.g., supporting tissue in the trabecular meshwork and in Schlemm's canal. The flow facilitated by the presence of the ocular implant may include axial flow along Schlemm's canal, flow into Schlemm's canal from the anterior chamber of the eye, and flow leaving Schlemm's canal via the outlets that communicate with the canal.

After exiting Schlemm's canal via the outlets, aqueous humor enters the venous blood stream and is carried along with the venous blood leaving the eye. The pressure of the venous system tends to be around 5-10 mm Hg above atmospheric pressure. Accordingly, the venous system provides a pressure backstop which assures that the pressure in the anterior chamber of the eye remains above atmospheric pressure.

The invention provides an ocular implant adapted to reside at least partially in a portion of Schlemm's canal of an eye. The ocular implant has a body extending in a curved volume whose longitudinal axis forms an arc of a circle, and a plurality of open areas and strut areas formed in the body, the open areas extending over more than 50% of a surface defining the curved volume, the strut areas surrounding the open areas, the body having a diameter of between 0,127 mm (0.005 inches) and 1,02 mm (0.04 inches):

In some embodiments, the open areas are formed in a first longitudinal section extending along the curved volume. This longitudinal section may include the largest radius portion of the curved volume. The open areas of the implant may also include a plurality of openings formed on a second longitudinal section of the implant body disposed, e.g., opposite the first longitudinal section. In addition, there may be spine sections disposed between the openings formed on the second longitudinal section.

In some embodiments, the strut areas extend axially and circumferentially around the body from one side of the first longitudinal section to the other side of the first longitudinal section. Some of the open areas may be formed between the strut areas.

In some embodiments, the implant is formed from shape memory material in a shape approximately equal to the curved volume. The curved volume of the implant may extend through a 60°-180° arc of a circle. In some embodiments, material coverage within the curved volume in circular cross-sections perpendicular to the longitudinal axis is less than 50% over greater than 90% of the implant.

In some embodiments, the implant has an inlet portion disposed at one end of the body in fluid communication with the body and extending inward from the circle arc. The inlet portion may extend at a 90° angle from a tangent drawn from a connection point of the inlet portion to the body. In some embodiments, the inlet portion has a length greater than the diameter of the body. The inlet portion may be formed, e.g., as a coil, a channel with at least one open longitudinal section, etc. in fluid communication with the body of the implant. The inlet portion may also extend along the same circle arc as the body.

In some embodiments, the implant may have a blunt tip disposed at one end, and there may be a lumen formed through the blunt tip.

In some embodiments, a therapeutic agent may deposited on the body of the implant. The therapeutic agent may be an anti-glaucoma drug such as a prostaglandin analog (e.g., latanprost).

The implant of the invention may have a variety of features. In some embodiments, the implant body has circumferential material coverage in cross-sections perpendicular to the longitudinal axis that is less than 360 degrees over an entire length of the body. The body may define an elongate slot that opens radially outward when the body is unrestrained.

In some embodiments, the body has a plurality of pairs of tissue supporting frames and a spine attached to each adjacent pair of tissue supporting frames. Each spine may have a shape that is symmetric about the first plane, and each tissue supporting frame may have a shape that is symmetric about the first plane. In some embodiments, the spines are longitudinally aligned with one another.

In some embodiments, each spine has a first minor side, a first major side, a second minor side, and a second major side; with each spine having a thickness extending between at least one point on each major side and at least one point on the second major side; each spine having a width extending between at least one point on each minor side and at least one point on the second minor side; and an aspect ratio of the width to the thickness being greater than about 2. In some embodiments, the first major side and the second major side are opposite one another. Also, the first major side may have a concave surface, and the second major side may have a convex surface. The concave surface and the convex surface may be concentric.

In some embodiments, each tissue supporting frame has at least a first strut and a second strut. Each strut may follow, e.g., a circumferentially undulating path while extending longitudinally between a first spine and a second spine.

In some embodiments, each strut has a first minor side, a first major side, a second minor side, and a second major side. In such embodiments, each strut has a thickness extending between at least one point on the first major side and at least one point on the second major side; a width extending between at least one point on the first minor side and at least one point on the second minor side; and an aspect ratio of the width to the thickness being greater than about 2. In some embodiments, the first major side and the second major side are opposite one another. Also, the first major side may have a concave surface, and the second major side may have a convex surface. The concave surface and the convex surface may be concentric.

In some embodiments, the body of the ocular implant exhibits superelastic properties. The body may be made of nickel and titanium in appropriate proportions, such as, e.g., wherein the weight percent of nickel is between about 29.5 and about 50.5 weight percent and the weight percent of titanium is between about 29.5 and about 50.5 weight percent, based upon the total weight percent of the alloy.

In some embodiments of the ocular implant, the body extends through an arcuate range between about 60 degrees and about 180 degrees. A therapeutic agent may be deposited on the implant body in some embodiments. The therapeutic agent may be an anti-glaucoma drug, such as a prostaglandin analog (e.g., latanprost).

A method of treating a human eye is described, including the steps of: inserting an implant into Schlemm's canal of a human eye, the implant having a first flexibility when bent along a first plane and a second flexibility different from the first flexibility when bent along a second plane that intersects the first plane and is not coincident with the first plane; and supporting tissue forming Schlemm's canal with the implant.

The implant has an elongate slot, the method further including the step of orienting the elongate slot radially outward within Schlemm's canal. The orienting step may be performed at least in part by permitting the implant to self-orient with the canal. A step of providing fluid communication between an anterior chamber and the canal through the implant may also be provided.

A method of treating glaucoma including the following steps is described: supporting tissue forming Schlemm's canal in an eye with an implant extending at least partially in the canal along an axial length within the canal; and contacting with the implant less than 50% of the tissue forming the canal along the axial length. The implant may have open areas separated by spine areas along a first longitudinal section, in which case the supporting step includes the step of orienting the first longitudinal section openings toward a trabecular mesh portion of the canal. The supporting step may also include the step of orienting a second longitudinal section of the implant which is at least 90% open opposite to the first longitudinal section within the canal.

The supporting step may include the step of supporting with the implant tissue extending approximately 60°-180° around the canal.

The method may include the step of providing fluid communication between an anterior chamber and the canal through the implant, such as by engaging trabecular mesh tissue with the implant.

The supporting step may include the step of supporting the tissue with the implant such that material coverage of tissue by the implant in cross-sections of the implant perpendicular to a longitudinal axis of the canal is less than 50% over greater than 90% of the axial length of the implant.

In some patients, Schlemm's canal may have become compartmentalized. When this is the case, Schlemm's canal becomes a series of small compartments separated by discontinuities or partitions. As the ocular implant is advanced into Schlemm's canal, the distal tip of the ocular implant penetrates the discontinuities/partitions. This penetrating action re-establishes fluid communication between adjacent compartments. The body of the ocular implant facilitates flow across the partitions by remaining in Schlemm's canal after fluid communication has been re-established.

Some exemplary ocular implants disclosed herein include a blunt tip having a generally rounded shape. For example, the blunt tip may have a generally hemispherical shape. The generally rounded shape of the blunt tip may increase the likelihood that the body of the ocular implant will track Schlemm's canal as the ocular implant is advanced into the canal during an implant procedure.

Some exemplary ocular implants disclosed herein include an inlet portion that is shaped and sized to extend through the trabecular meshwork of the eye. This inlet portion may provide a flow path between the anterior chamber and Schlemm's canal. After entering Schlemm's canal, aqueous humor may flow between a proximal portion of the ocular implant and an intermediate portion of the ocular implant. The intermediate portion of the ocular implant may be conceptualized as a manifold that distributes the aqueous humor along a portion of Schlemm's canal. A plurality of outlets may be located along the length of this portion of Schlemm's canal. When this is the case, the presence of the ocular implant in Schlemm's canal facilitates the flow of aqueous humor through those outlets.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:

Figure 1 is a plan view showing a portion of an eye.

Figure 2 is an enlarged plan view of a portion of the eye shown in the previous figure.

Figure 3 is a top plan view showing an intermediate portion of an exemplary ocular implant.

Figure 4 is a sideplan view of the ocular implant shown in the previous figure.

Figure 5 is a lateral cross-sectional view of the ocular implant shown the previous figure.

Figure 6 is an additional lateral cross-sectional view of the ocular implant shown the previous figure.

Figures 7A, 7B, and 7C are side, bottom and top plan views (respectively) illustrating an exemplary ocular implant.

Figures 8A, 8B, and 8C are additional, larger side, bottom and top plan views (respectively) of the exemplary ocular implant shown in Figures 7A, 7B, and 7C.

Figure 9 is an additional side plan view illustrating the ocular implant shown in the previous figure.

Figure 10 is a top plan view illustrating the ocular implant shown in the previous figure.

Figure 11 is a perspective view of an exemplary ocular implant.

Figure 12 is a plan view of an additional exemplary ocular implant.

Figures 13A, 13B, and 13C are side, bottom and top plan views (respectively) illustrating another exemplary ocular implant.

Figure 14 is a perspective view of an ocular implant.

Figure 15 is a side view of the ocular implant of Figure 14.

Figure 16 is a perspective view of yet another ocular implant.

Figure 17 is a perspective view of still another ocular implant.

Figure 18 shows the ocular implant of Figures 11 and 12 in place within a patient's eye.

Figure 19 shows the ocular implant of Figures 14 and 15 in place within a patient's eye.

Figure 20 is a perspective view depicting a portion of a human eye and a portion of an ocular implant disposed in Schlemm's canal.

Figure 21 is an enlarged perspective view showing a portion of the implant of Figure 20.

Figure 22 is a perspective view showing a volume defined by the body of the ocular implant of Figures 20 and 21.

Figure 23 is a perspective view showing a first plane intersecting the body of an ocular implant.

Figure 24 is a perspective view showing a bending moment being applied to an ocular implant.

Figure 25 is a plan view of the implant shown in Figure 24 but in the absence of any bending moment.

Figure 26A is a lateral cross-sectional view of the ocular implant of Figure 25 taken along section line A-A of Figure 25.

Figure 26B is a lateral cross-sectional view of the ocular implant of Figure 25 taken along section line B-B of Figure 25.

Figure 27 is an enlarged cross-sectional view of the ocular implant of Figure 25 taken along section line B-B of Figure 25.

Figure 28 is an enlarged cross-sectional view of the ocular implant of Figure 25 taken along section line A-A of Figure 25.

Figure 29 is a plan view showing an ocular implant according to another embodiment of the invention having a longitudinal radius of curvature that varies along its length.

Figure 30 is a perspective view showing an ocular implant according to yet another embodiment of the invention that has substantially no radius of curvature.

Figure 31 is an isometric view showing a body that may be used to form an ocular implant in accordance with one exemplary embodiment of the invention.

Figure 32 is an isometric view of the body shown in the previous figure.

Figure 33A is a plan view showing a portion of the ocular implant shown in the previous figure.

Figure 33B is a lateral cross-sectional view of the ocular implant shown in Figure 33A taken along section line B-B.

Figure 33C is a lateral cross-sectional view of the ocular implant of Figure 33A taken along section line C-C.

Figure 34 is an isometric view showing a portion of the ocular implant shown in the previous figure.

Figure 35 is an enlarged plan view showing a portion of the ocular implant shown in Figure 34.

Figure 36A through 36E are lateral cross-sectional views of the ocular implant shown in Figure 35 taken along section lines D-D, E-E, F-F, G-G, and H-H, respectively.

Figure 37 shows a plurality of cylindrical coordinate values corresponding with the cross-sectional views shown in the Figures 36A-E.

Figure 38 is an isometric view of an ocular implant in accordance with an additional exemplary embodiment of the invention.

Figure 39 shows an ocular implant of the invention in place within a human eye.

Figure 40 is an enlarged plan view showing a portion of the eye shown in the previous figure.

### DETAILED DESCRIPTION OF THE INVENTION

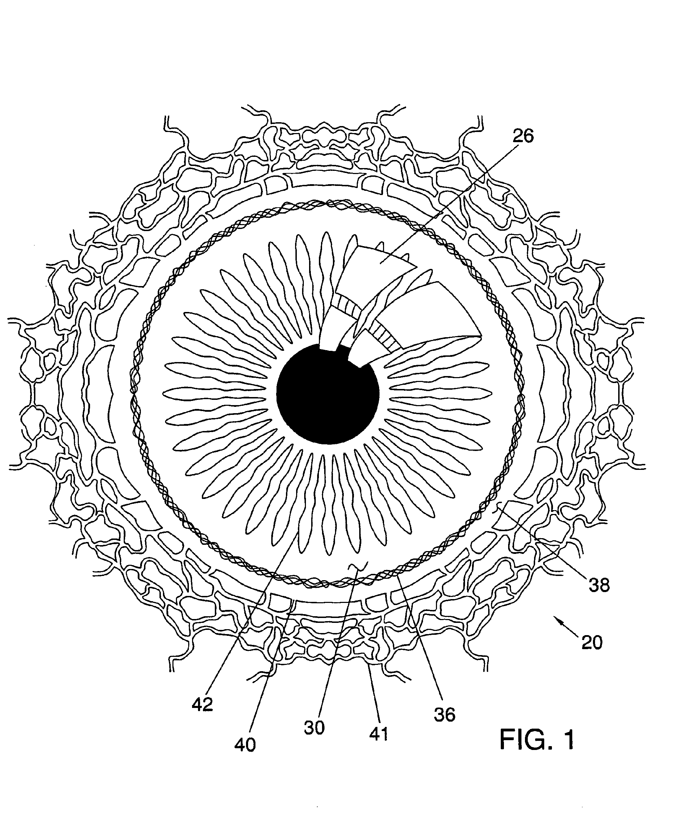

Figures 1-3 are views depicting a portion of a human eye 20. Eye 20 can be conceptualized as a fluid filled ball having two chambers. Sclera 22 of eye 20 surrounds a posterior chamber 24 filled with a viscous fluid known as vitreous humor. Cornea 26 of eye 20 encloses an anterior chamber 30 that is filled with a fluid know as aqueous humor. The cornea 26 meets the sclera 22 at a limbus 28 of eye 20. A lens 32 of eye 20 is located between anterior chamber 30 and posterior chamber 24. Lens 32 is held in place by a number of ciliary zonules 34.

Whenever a person views an object, he or she is viewing that object through the cornea, the aqueous humor, and the lens of the eye. In order to be transparent, the cornea and the lens can include no blood vessels. Accordingly, no blood flows through the cornea and the lens to provide nutrition to these tissues and to remove wastes from these tissues. Instead, these functions are performed by the aqueous humor. A continuous flow of aqueous humor through the eye provides nutrition to portions of the eye (e.g., the cornea and the lens) that have no blood vessels. This flow of aqueous humor also removes waste from these tissues.

Aqueous humor is produced by an organ known as the ciliary body. The ciliary body includes epithelial cells that continuously secrete aqueous humor. In a healthy eye, a stream of aqueous humor flows out of the eye as new aqueous humor is secreted by the epithelial cells of the ciliary body. This excess aqueous humor enters the blood stream and is carried away by venous blood leaving the eye. The structures that drain aqueous humor from anterior chamber 30 include Schlemm's canal 38 and a large number of veins 40.

In Figure 1, Schlemm's canal 38 can be seen encircling iris 42. Aqueous humor exits anterior chamber 30 and enters Schlemm's canal 38 by flowing through a trabecular mesh 36. Aqueous humor exits Schlemm's canal 38 by flowing through a number of outlets 40. After leaving Schlemm's canal 38, aqueous humor travels through veins 41 and is absorbed into the blood stream. Schlemm's canal typically has a non-circular cross-sectional shape whose diameter can vary along the canal's length and according to the angle at which the diameter is measured. In addition, there may be multiple partial pockets or partial compartments (not shown in these figures) formed along the length of Schlemm's canal. The shape and diameter of portions of Schlemm's canal and the existence and relative location of partial pockets or compartments may limit or prevent fluid flow from one point of Schlemm's canal to another. Hence, each outlet 40 from Schlemm's canal may drain only a portion of Schlemm's canal.

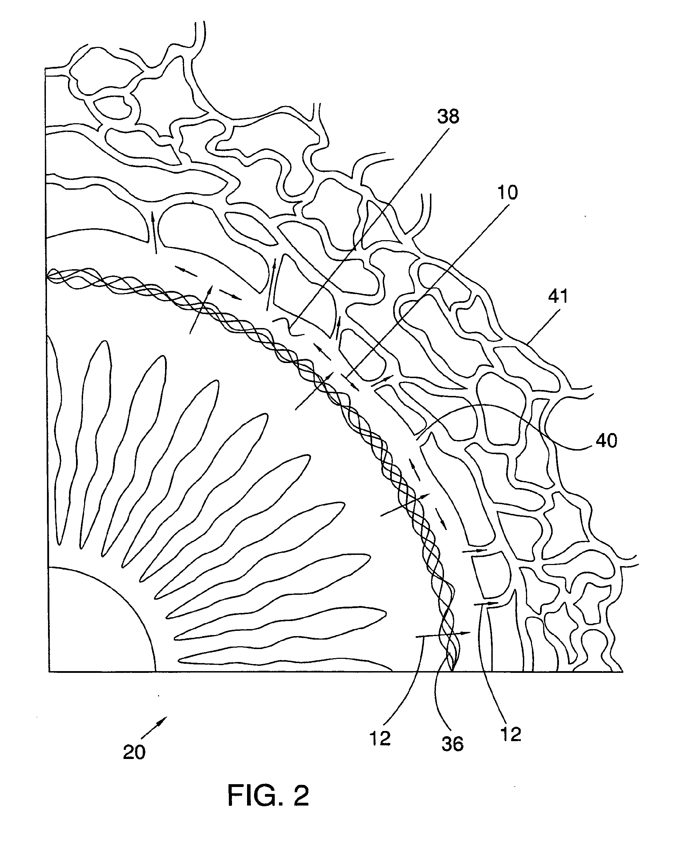

Figure 2 is an enlarged plan view of a portion of eye 20 shown in the previous figure. The flow of aqueous humor in a healthy eye 20 is illustrated using arrows in Figure 2. In Figure 2, aqueous humor flowing through trabecular mesh 36 and into Schlemm's canal 38 is represented by a number of lateral flow arrows 12. The flow of aqueous humor along the length of Schlemm's canal is illustrated using a number of axial flow arrows 10.

With reference to Figure 2, it will be appreciated that a number of outlets 40 communicate with Schlemm's canal 38. In Figure 2, the flow of aqueous humor exiting Schlemm's canal 38 and flowing through outlets 40 is illustrated with additional lateral flow arrows 12. After leaving Schlemm's canal 38, aqueous humor travels through veins 41 and is absorbed into the blood stream.

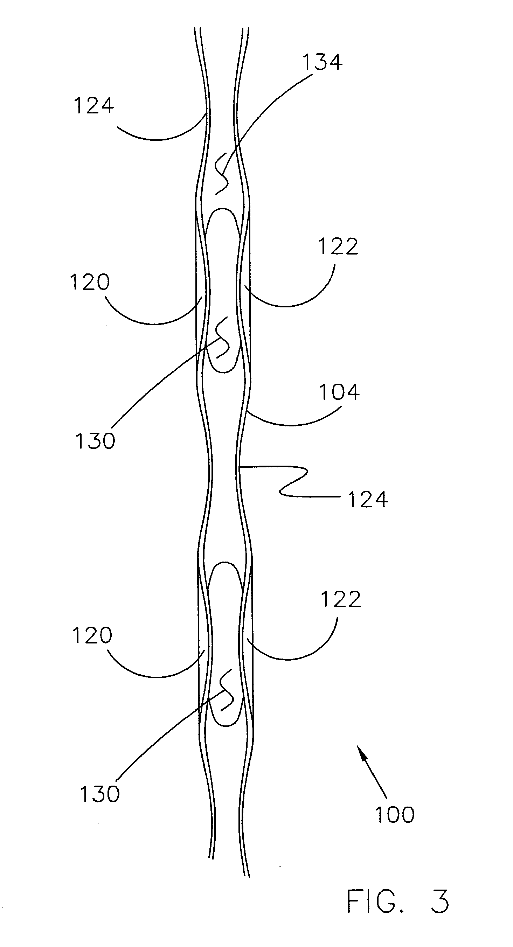

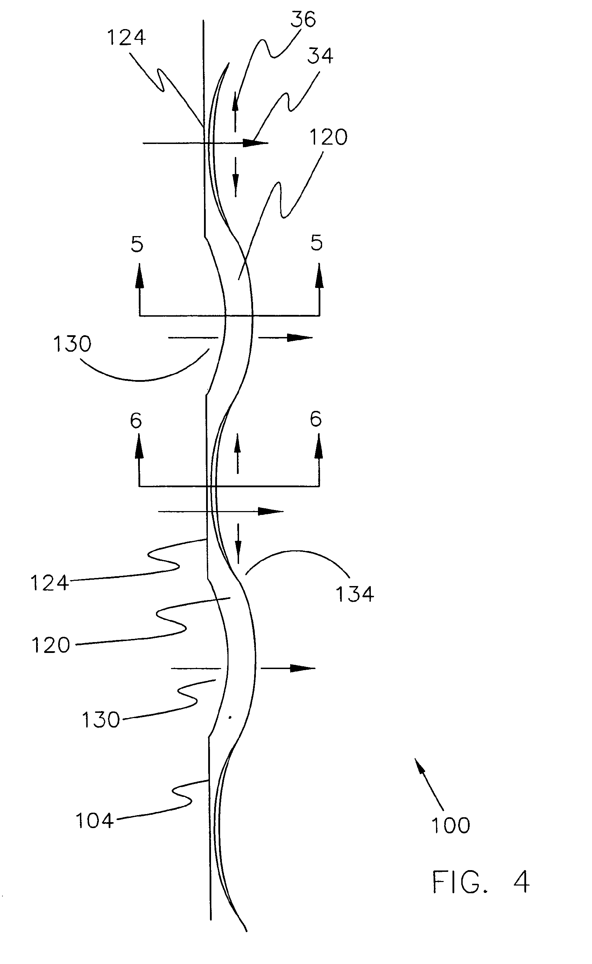

Figures 3 and 4 are top and side views showing an intermediate portion of an exemplary ocular implant 100. Ocular implant 100 may be inserted into Schlemm's canal, the trabecular meshwork and the anterior chamber to facilitate the outflow of aqueous humor from the anterior chamber. This flow may include axial flow along Schlemm's canal, flow from the anterior chamber into Schlemm's canal, and flow leaving Schlemm's canal via outlets communicating with Schlemm's canal. When in place within the eye, ocular implant 100 will support trabecular mesh tissue and Schlemm's canal tissue and will provide for improved communication between the anterior chamber and Schlemm's canal (via the trabecular meshwork) and between pockets or compartments along Schlemm's canal.

Ocular implant 100 of Figures 3 and 4 comprises a body 104 having an outer surface 106. Body 104 of ocular implant 100 has a plurality of pairs of struts 120 and 122 separated by spine sections 124. The struts and spines define an open channel 134 whose open side lies along one longitudinal section of the implant body. A plurality of openings 130 are formed between the struts 120 and 122 on a longitudinal section of the implant opposite to the open side of channel 134. While in this embodiment the openings 130 are 180° from the open side of channel 134, in other embodiments openings 130 may be disposed 140°-150° the open side of channel 134. The diameter of body 104 is selected to support the tissue of Schlemm's canal without stretching it and is preferably in the range of 0,127 mm (0.005 inches) to 1,02 mm (0.04 inches), most preferably in the range of 0,127 mm (0.005 inches) to 0,508 mm (0.02 inches)

As shown in these figures, aqueous humor may flow axially down open channel 134 (as shown by arrows 36 in Figure 4) or out of the implant through the opening of open channel 134 (first passing, e.g., through openings 130 and/or along the channel 134) as represented by lateral flow arrows 34. When implanted, body 104 of implant 100 preferably extends 60°, 90°, 150° or 180° around the circle formed by Schlemm's canal. The arrangement of struts, open areas and spine areas along implant 100 supports the tissue of Schlemm's canal with a minimum amount of material. In the embodiment shown in Figures 3 and 4, for example, the open areas extend over more than 50% of a hypothetical surface covering the volume of the portion of the implant lying within Schlemm's canal. This combination of features helps aqueous humor flow between any pockets or compartments formed within Schlemm's canal and, therefore, between the anterior chamber and the outlets from Schlemm's canal to the venous system.

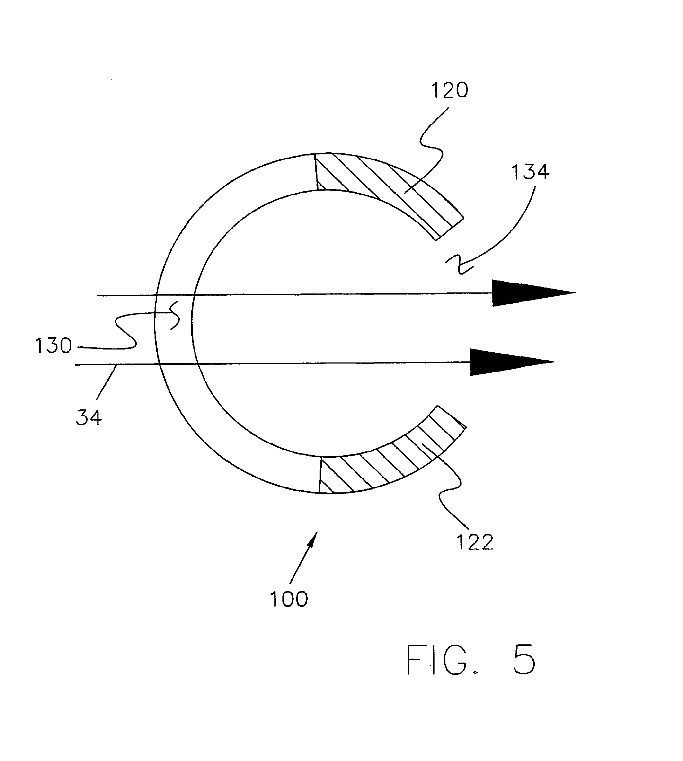

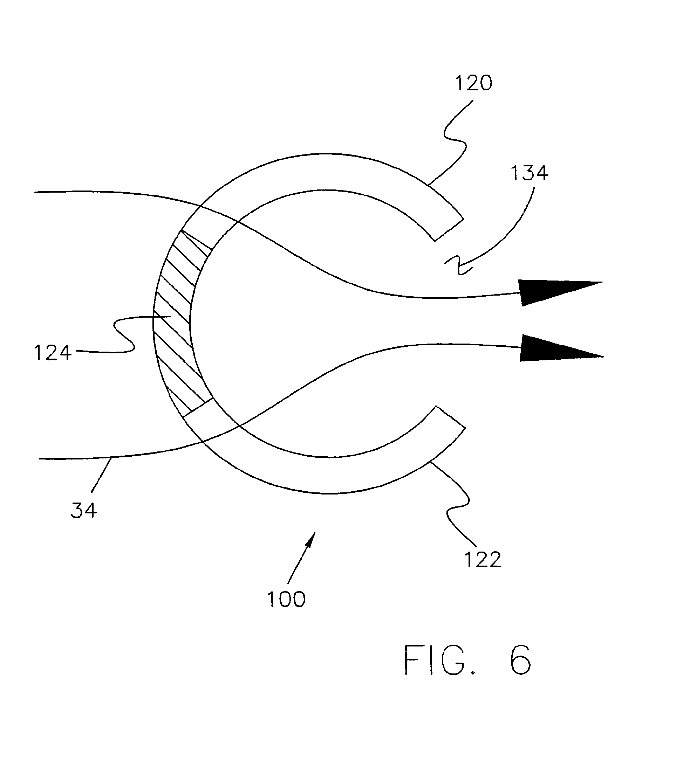

Figure 5 is a lateral cross-sectional view of ocular implant 100 taken along line 5-5 shown in Figure 4, and Figure 6 is a lateral cross-sectional view of ocular implant 100 taken along line 6-6 shown in Figure 4. There are normally many flow paths from the anterior chamber through the trabecular meshwork into Schlemm's canal. Aqueous humor may therefore flow into channel 134 in body portion 104 of implant 100 from the trabecular meshwork through one or more openings 130 and/or around the struts 120/122 and spines 124. Thus, in Figure 5, aqueous humor flowing past a spine area 124 is illustrated with lateral flow arrows 34 and ,in Figure 6, aqueous humor flowing between first strut area 120 and second strut area 122 is illustrated using lateral flow arrows 34.

Figures 5 and 6 also illustrate another unique feature of implant 100: The arrangement of struts, openings and spine areas ensures that material coverage of Schlemm's canal in virtually any lateral cross-section of the implant and canal is less than 50%. This material coverage relationship hold true for over 90% of the implant's length.

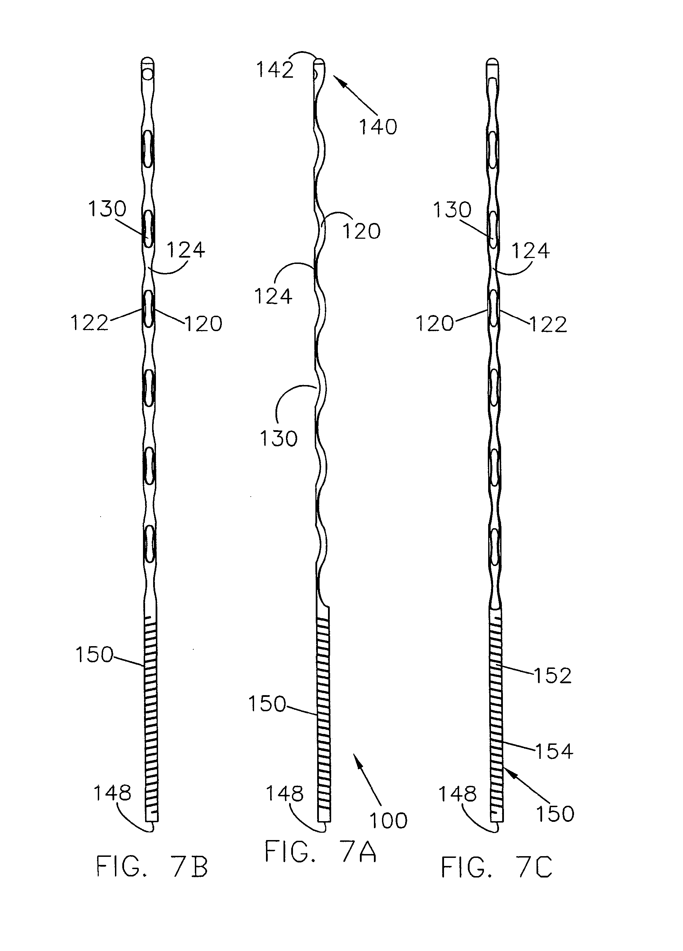

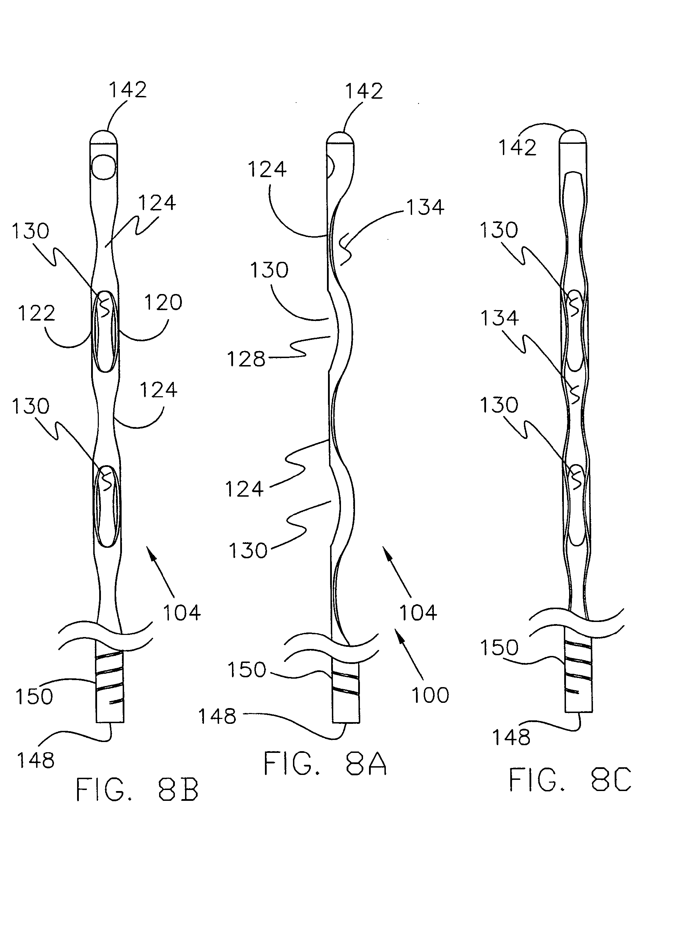

In some embodiments, in addition to a Schlemm's canal portion as described above, the ocular implant also includes at least one optional inlet portion adapted to be at least partially disposed in the anterior chamber of the eye. The inlet portion is configured to support trabecular mesh tissue and to permit aqueous humor to flow from the anterior chamber into the open channel of the implant within Schlemm's canal. Figures 7A-C and 8A-C illustrate an exemplary ocular implant 100 with an optional inlet region 150 in addition to a plurality of struts 120, 122, openings 130 and spine areas 124 substantially the same as the previous embodiment. In the embodiment of Figures 7 and 8, inlet region 150 of ocular implant 100 comprises a coil. Coil 150 comprises a plurality of turns 152 that are defined by a generally helical slot 154. Coil 150 may be bent so as to project through the trabecular mesh into the anterior chamber while the remainder of the device lies within Schlemm's canal. Aqueous humor can flow into the inlet region through an open end 148 and through slot 154.

In some embodiments, the ocular implant may have an optional blunt tip for use in facilitating atraumatic delivery of the device into Schlemm's canal. As shown in Figures 7 and 8, distal portion 140 of ocular implant 100 comprises a blunt tip 142. In some useful embodiments of ocular implant 100, blunt tip 142 has a generally rounded shape. In the embodiment shown in Figures 7 and 8, blunt tip 142 has a generally hemispherical shape.

In the embodiment of Figures 7 and 8, body 104 of ocular implant 100 is pictured assuming a generally straight shape. Embodiments of ocular implant 100 are possible in which body 104 has a generally curved resting shape.

Ocular implant 100 may be used in conjunction with a method of treating a patient. Some such methods may include the step of inserting a core member into a lumen defined by ocular implant 100. The core member may comprise, for example, a wire or tube. The distal end of the ocular implant may be inserted into Schlemm's canal. The ocular implant and the core member may then be advanced into Schlemm's canal until the ocular implant has reached a desired position. The core member may then be withdrawn from the ocular implant.

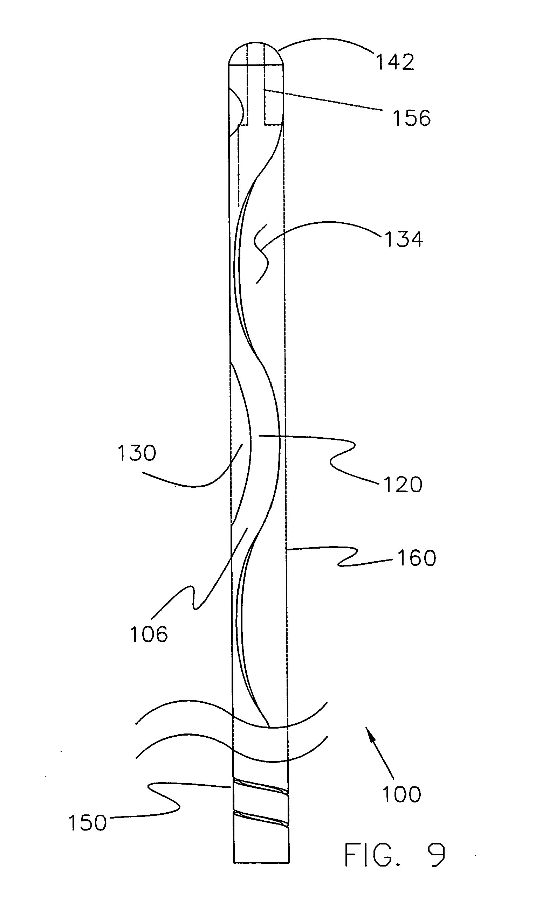

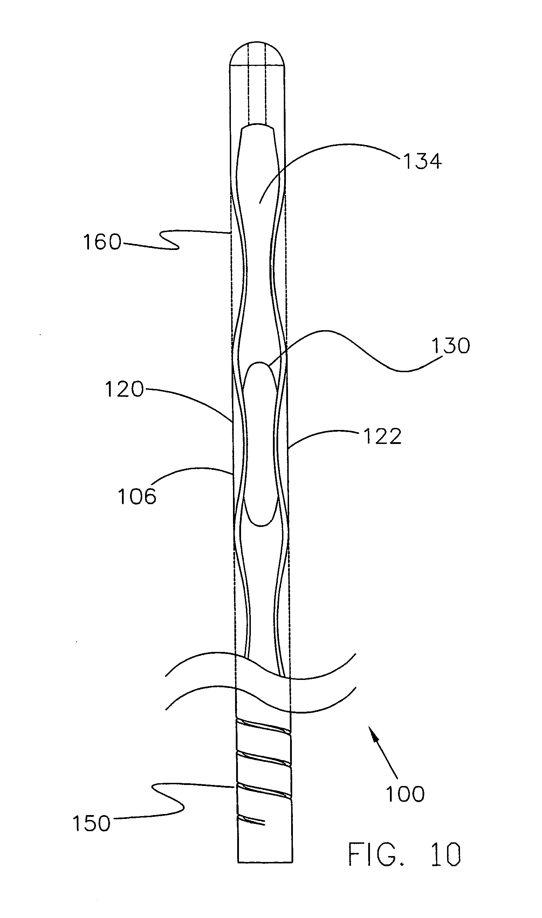

Figures 9 and 10 show another embodiment of an ocular implant 100 similar to that of Figures 7 and 8. With reference to Figures 9 and 10, a lumen 156 is formed in blunt tip 142. This lumen may be used to inject a contrast medium through the blunt tip during implantation of the implant into the patient's eye. Lumen 156 may also be used to inject a visco-elastic medium in front of the implant to part tissue as the implant moves into Schlemm's canal.

A dotted line 160 in Figures 9 and 10 indicates a cylindrical envelope surrounding implant 100. In some embodiments, the open areas of ocular implant 100 (made up of openings 130 and the open portion of open channel 134) extend over more than 50% of cylindrical surface 160.

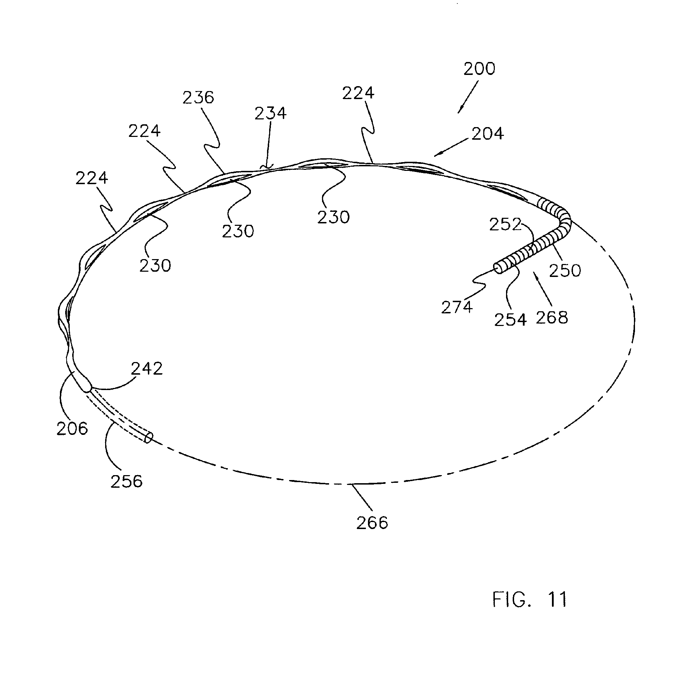

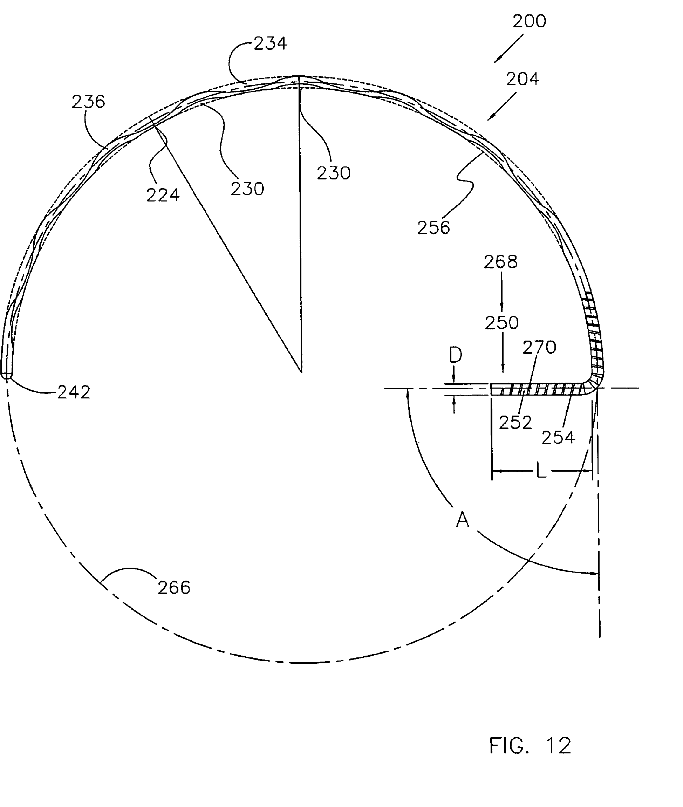

Figures 11 and 12 show an additional exemplary ocular implant 200 according to the invention. In the embodiment of Figures 11 and 12, no external forces are acting on ocular implant 200, and ocular implant 200 is free to assume a generally curved resting shape in which its longitudinal axis forms an arc of a circle 266, as depicted in Figures 11 and 12. In some useful embodiments of ocular implant 200, a relatively stiff core may be placed in the ocular implant 200 to cause it to assume a generally straight shape during delivery.

As shown in Figures 11 and 12, implant 200 has a plurality of openings 230 along a longitudinal section on a shorter radius side of the body, as well as an open channel 234 facing radially outward on a longitudinal section forming the largest radius portion of the body. As in the prior embodiments, implant 200 also has a plurality of struts 236 and spine areas 224 formed in the body portion 204 of the implant. As shown, the open areas (including the openings 230 and the open portion of channel 234) extend over more than 50% of the surface of a hypothetical cylinder 256 surrounding the implant 200. In addition, material coverage of Schlemm's canal in cross-sections taken over 90% of the length of implant 200 is less than 50%, as in the previous embodiment.

Ocular implant 200 of Figures 11 and 12 includes an inlet portion 268 extending inward from circle 266. Inlet portion 268 of ocular implant 200 comprises a coil 250 having a plurality of turns 252 that are defined by a generally helical slot 254. An inlet 274 is formed in one end of inlet portion 268. Inlet portion 268 will extend through the trabecular meshwork into the anterior chamber of the eye when body portion 204 lies in Schlemm's canal.

Ocular implant 200 of Figures 11 and 12 includes a blunt tip 242 with a generally rounded shape. The generally rounded shape of blunt tip 242 may increase the likelihood that body 204 will track Schlemm's canal as ocular implant 200 is advanced into the canal during an implant procedure.

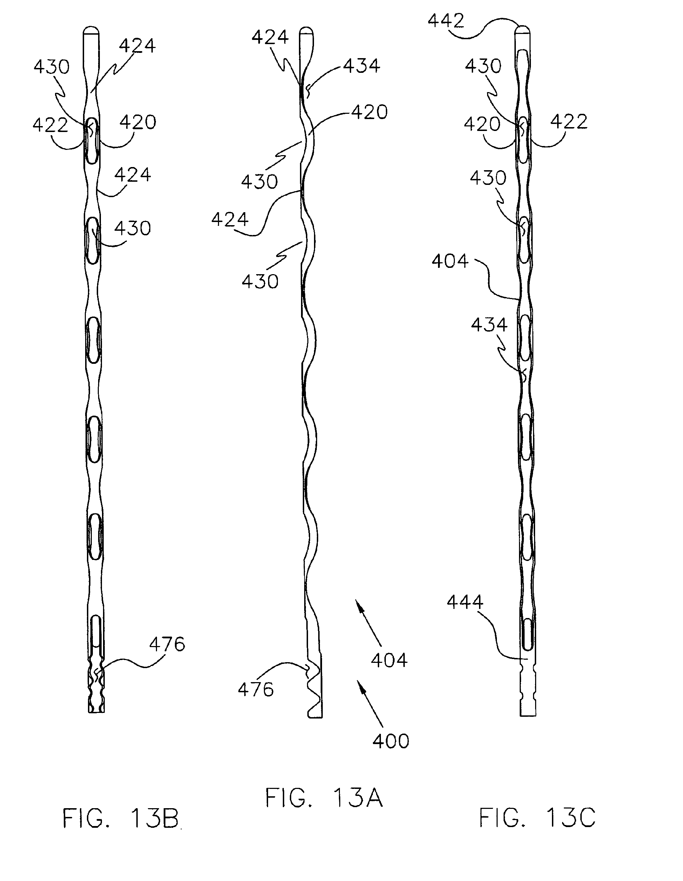

As shown in Figures 11 and 12, ocular implant 200 extends through a 180° arc of circle 366. Other implant sizes are possible, of course, such as implants extending 60°, 90° and 150° around a circle. As shown in Figure 12, inlet portion 268 is shown extending at an angle A from a tangent line T. In the embodiment of Figure 13, angle A is about 90 degrees. Inlet portion 268 has a length L and body 204 of ocular implant 300 has a diameter D. In the embodiment of Figure 12, length L is greater than diameter D. As in the other embodiments, the diameter can range from 0.005 inches to 0.04 inches, preferably from 0.005 inches to 0.02 inches, in order to lie within and support Schlemm's canal.

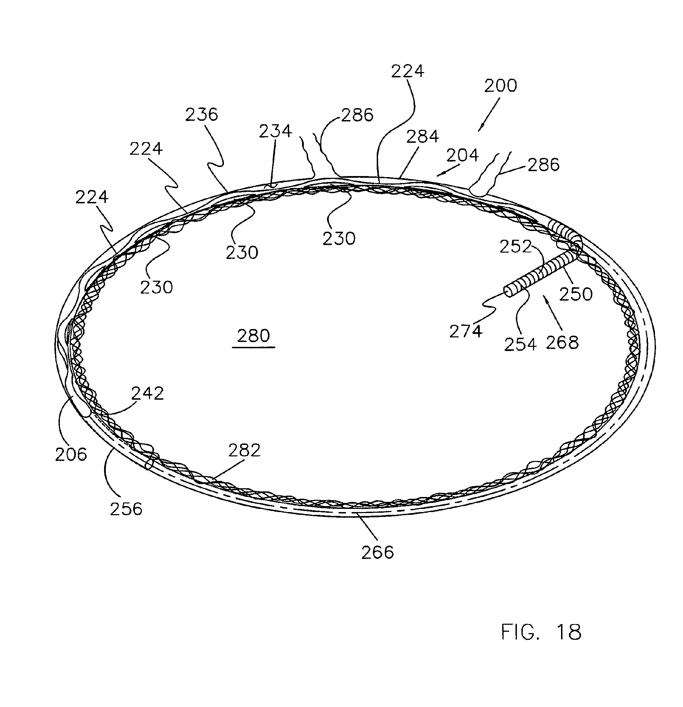

Figure 18 shows the implant of Figures 11 and 12 in place within a patient's eye. The body portion (including the plurality of strut pairs 236, openings 230, open channel 234, spine areas 224 and the blunt tip 242) lie within and support the walls of Schlemm's canal 284. The openings 230 are oriented at least partially toward the trabecular meshwork 282, and the open portion of open channel 234 is oriented on the largest radius portion of the canal facing openings 286 from Schlemm's canal into the venous system (not shown). As shown, the body of the implant extends approximately 180° around the canal. The inlet portion 250 of the implant extends through the trabecular meshwork 282 into the anterior chamber 280 so that the inlet 274 and spiral slot 254 are in fluid communication with the aqueous humor within the anterior chamber.

Figures 13A-C show an additional exemplary ocular implant 400. As in the embodiments shown above, ocular implant 400 comprises a body 404 having a plurality of openings 430, an open channel 434, pairs of struts 420 and 422, and spine areas 424. As in the earlier embodiments, the open areas (including the openings 430 and the open portion of channel 434) extend over more than 50% of a hypothetical cylinder surrounding the body portion 404 of implant 400, and material coverage of Schlemm's canal in cross-sections taken over 90% of the length of the implant 400 is less than 50%. A blunt tip 442 is also provided, as in the earlier embodiments.

The inlet portion 450 of the implant differs from prior embodiments, however. Inlet portion 450 is formed as an open channel 476. When the body portion 404 of the implant is disposed in Schlemm's canal and inlet portion 150 projects through the trabecular meshwork into the anterior chamber, aqueous humor can flow into the implant through the open channel 476 and then into the body portion 404 within Schlemm's canal. The open nature of inlet portion 450 reduces the speed with which aqueous humor will flow into the implant, thereby reducing potential damage to adjacent tissue from suction forces associated with the flow.

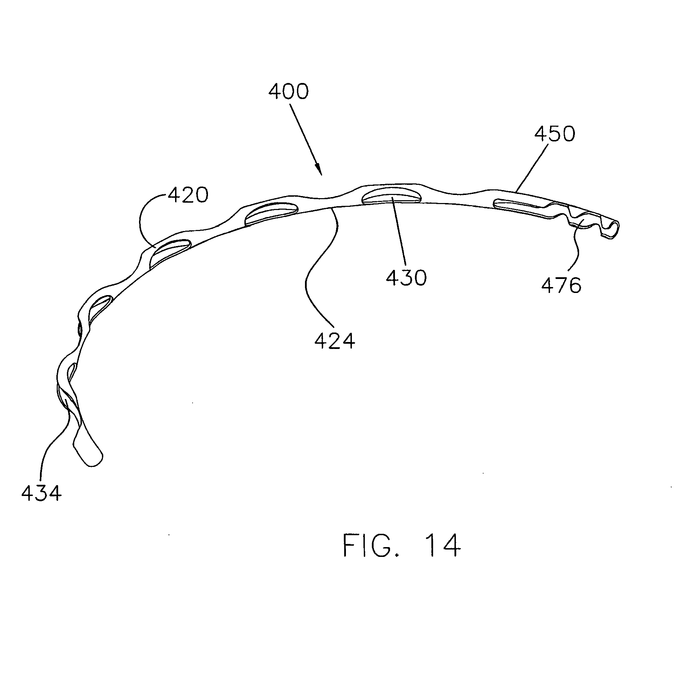

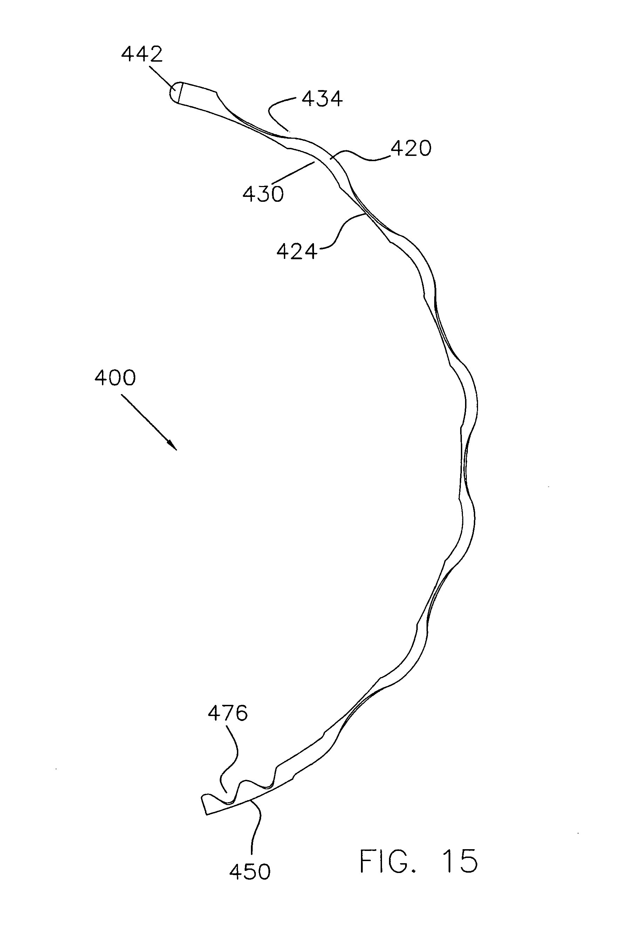

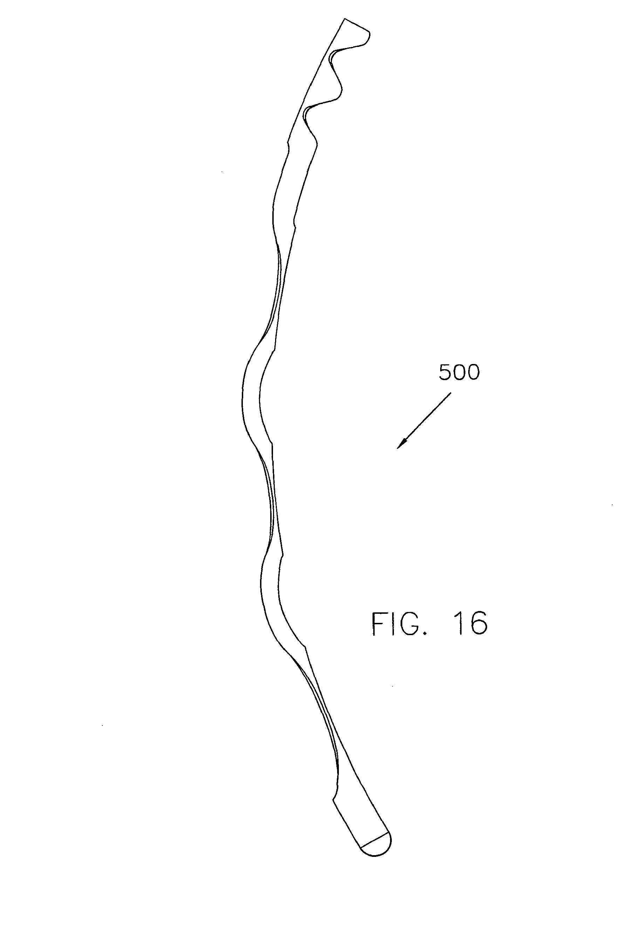

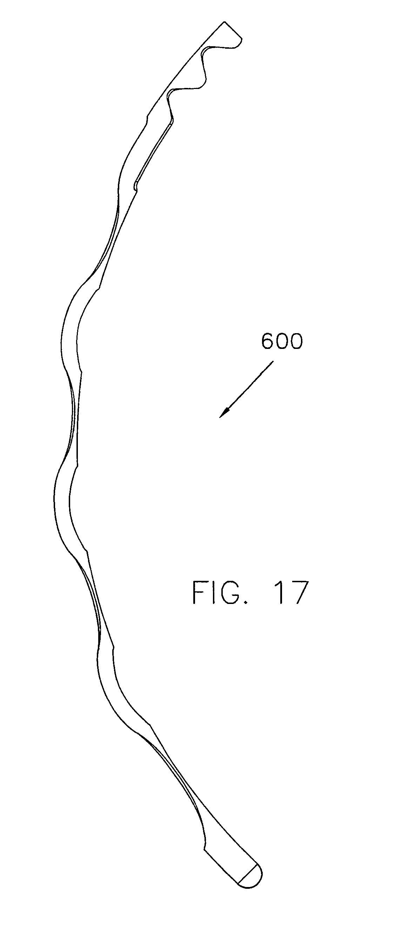

Figures 14 and 15 show embodiments similar to that of Figures 13 in which the implant 400 has an at rest shape in the form of an arc of a circle. As in the earlier embodiments, the implant may extend around any portion of the circle, such as 60°, 90°, 150° or 180°. For example, the implant of Figures 14 and 15 extends in a 150° arc, an implant 500 extending in a 60° arc is shown in Figure 16, and an implant 600 extending in a 90° arc is shown in Figure 17.

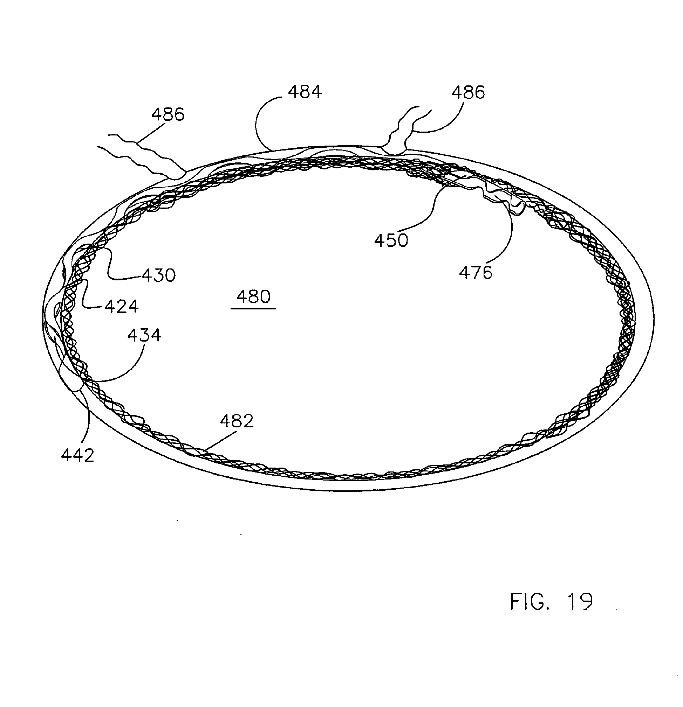

Unlike the embodiment shown in Figures 11 and 12, however, inlet portion 450 lies along the same circle arc as the rest of the implant. When inlet portion 450 is disposed in the anterior chamber (as shown in Figure 19) and the other portions of the implant lie in Schlemm's canal, the direction of axial flow of aqueous humor from inlet 450 into open channel 434 does not change as dramatically as in embodiments in which the inlet portion is at a 90° angle to the body portion of the implant.

Figure 19 shows the implant of Figures 14 and 15 in place within a patient's eye. The body portion (including the plurality of strut pairs 420, openings 430, open channel 434, spine areas 424 and the blunt tip 442) lie within and support the walls of Schlemm's canal 484. The openings 430 are oriented at least partially toward the trabecular meshwork 482, and the open portion of open channel 434 is oriented on the largest radius portion of the canal facing openings 486 from Schlemm's canal into the venous system (not shown). As shown, the body of the implant extends approximately 150° around the canal. The inlet portion 450 of the implant extends through the trabecular meshwork 482 into the anterior chamber 480 so that the open channel 476 of the inlet portion is in fluid communication with the aqueous humor within the anterior chamber.

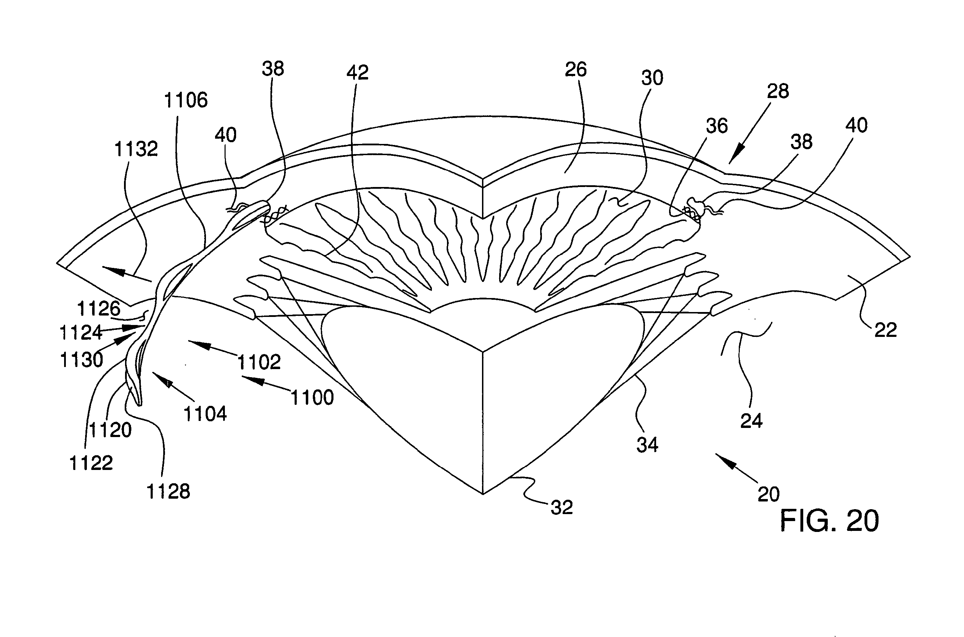

In Figure 20, an ocular implant 1100 is disposed in Schlemm's canal 38 of eye 20. Ocular implant 1100 has a body 1102 including a plurality of tissue supporting frames 1104 and a plurality of spines 1106. Body 1102 also includes a first edge 1120 and a second edge 1122 that define a first opening 1124. First opening 1124 is formed as a slot and fluidly communicates with an elongate channel 1126 defined by an inner surface 1128 of body 1102. With reference to Figure 20, it will be appreciated that first opening 1124 is disposed on an outer side 1130 of body 1102. Accordingly, channel 1126 opens in a radially outward direction 1132 via first opening 1124.

Ocular implant 1100 may be inserted into Schlemm's canal of a human eye to facilitate the flow of aqueous humor out of the anterior chamber. This flow may include axial flow along Schlemm's canal, flow from the anterior chamber into Schlemm's canal, and flow leaving Schlemm's canal via outlets communicating with Schlemm's canal. When in place within the eye, ocular implant 1100 will support trabecular mesh tissue and Schlemm's canal tissue and will provide for improved communication between the anterior chamber and Schlemm's canal (via the trabecular meshwork) and between pockets or compartments along Schlemm's canal. As shown in Figure 20, the implant is preferably oriented so that the first opening 1124 is disposed radially outwardly within Schlemm's canal.

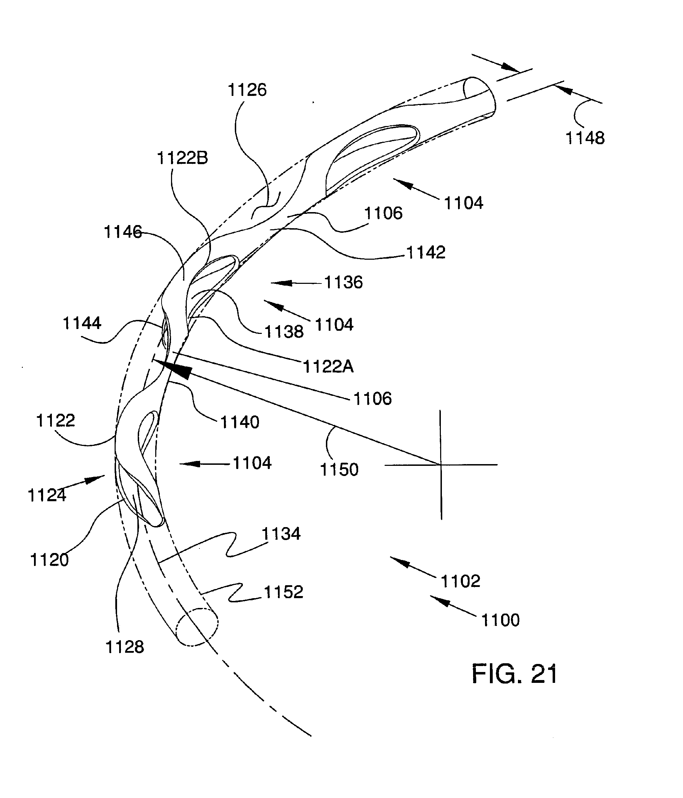

Figure 21 is an enlarged perspective view showing a portion of ocular implant 1100 shown in the previous figure. Ocular implant 1100 has a body 1102 that extends along a generally curved longitudinal axis 1134. Body 1102 has a plurality of tissue supporting frames 1104 and a plurality of spines 1106. As shown in Figure 21, these spines 1106 and frames 1104 are arranged in a repeating AB pattern in which each A is a tissue supporting frame and each B is a spine. In the embodiment of Figure 21, one spine extends between each adjacent pair of frames 1104.

The frames 1104 of body 1102 include a first frame 1136 of ocular implant 1100 that is disposed between a first spine 1140 and a second spine 1142. In the embodiment of Figure 21, first frame 136 is formed as a first strut 144 that extends between first spine 140 and second spine 142. First frame 136 also includes a second strut 146 extending between first spine 140 and second spine 142. In the exemplary embodiment of Figure 2, each strut undulates in a circumferential direction as it extends longitudinally between first spine 140 and second spine 142.

In the embodiment of Figure 21, body 1102 has a longitudinal radius 1150 and a lateral radius 1148. Body 1102 of ocular implant 1100 includes a first edge 1120 and a second edge 1122 that define a first opening 1124. First opening 1124 fluidly communicates with an elongate channel 1126 defined by an inner surface 1128 of body 1102. A second opening 1138 is defined by a second edge 1122A of a first strut 1144 and a second edge 1122B of a second strut 1146. First opening 1124, second opening 1138 and additional openings defined by ocular implant 1100 allow aqueous humor to flow laterally across and/or laterally through ocular implant 1100. The outer surfaces of body 1102 define a volume 1152.

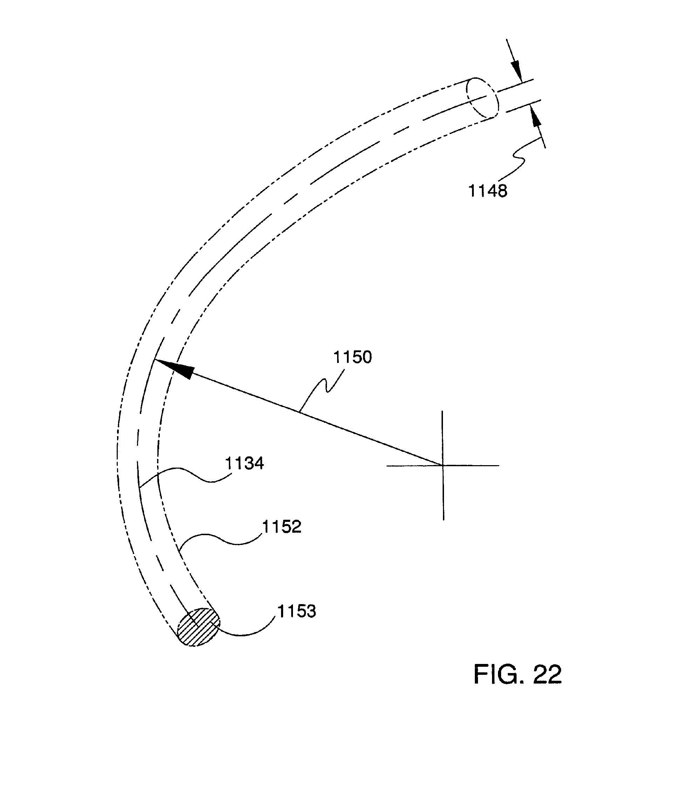

Figure 22 is an additional perspective view showing volume 1152 defined by the body of the ocular implant shown in the previous figure. With reference to Figure 22, it will be appreciated that volume 1152 extends along a generally curved longitudinal axis 1134. Volume 1152 has a longitudinal radius 1150, a lateral radius 1148, and a generally circular lateral cross section 1153.

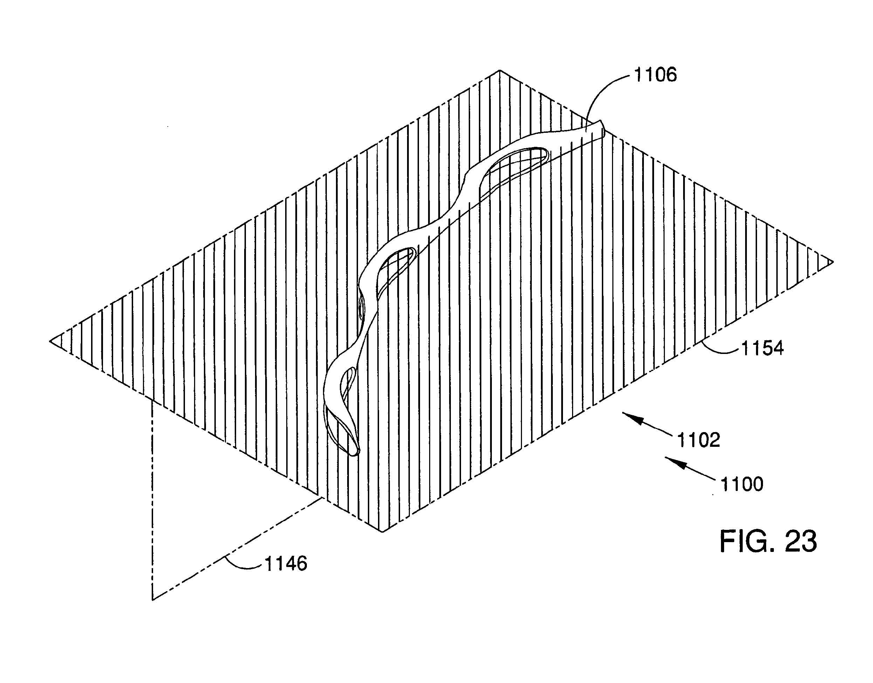

Figure 23 is a perspective view showing a first plane 1154 and a second plane 1155 that both intersect ocular implant 1100. In Figure 23, first plane 1154 is delineated with hatch marks. With reference to Figure 23, it will be appreciated that spines 1106 of body 1102 are generally aligned with one another and that first plane 1154 intersects all spines 1106 shown in Figure 23. In the embodiment of Figure 4, body 1102 of ocular implant 1100 is generally symmetric about first plane 1154.

In the embodiment of Figure 23, the flexibility of body 1102 is at a maximum when body 1102 is bending along first plane 1154, and body 1102 has less flexibility when bending along a plane other than first plane 1154 (e.g., a plane that intersects first plane 1154). For example, in the embodiment shown in Figure 23, body 1102 has a second flexibility when bending along second plane 1155 that is less than the first flexibility that body 1102 has when bending along first plane 1154.

Stated another way, in the embodiment of Figure 23, the bending modulus of body 1102 is at a minimum when body 1102 is bent along first plane 1154. Body 1102 has a first bending modulus when bent along first plane 1154 and a greater bending modulus when bent along a plane other than first plane 1154 (e.g., a plane that intersects first plane 1154). For example, in the embodiment shown in Figure 23, body 1102 has a second bending modulus when bent along second plane 1155 that is greater than the first bending modulus that body 1102 has when bent along first plane 1154.

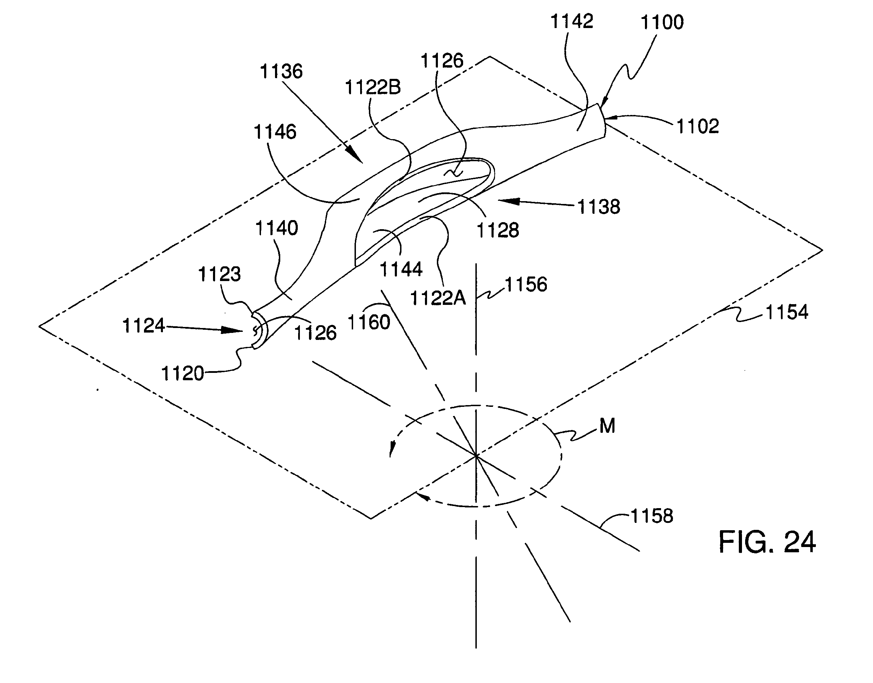

Figure 24 is an enlarged perspective view showing a portion of ocular implant 1100 shown in the previous figure. In the exemplary embodiment of Figure 24, a bending moment M is being applied to body 1102 of ocular implant 1100. Bending moment M acts about a first axis 1156 that is generally orthogonal to first plane 1154. A second axis 1158 and a third axis 1160 are also shown in Figure 24. Second axis 1158 is generally perpendicular to first axis 1156. Third axis 1160 is skewed relative to first axis 1156.

An inner surface 1128 of body 1102 defines a channel 1126. Body 1102 of ocular implant 1100 includes a first edge 1120 and a second edge 1123 that define a first opening 1124. Channel 1126 of ocular implant 1100 fluidly communicates with first opening 1124. A second opening 1138 is defined by a second edge 1122A of a first strut 1144 and a second edge 1122B of a second strut 1146. First opening 1124, second opening 1138 and additional openings defined by ocular implant 1100 allow aqueous humor to flow laterally across and/or laterally through ocular implant 1100.

As shown in Figure 24, ocular implant 1100 has a first spine 1140 and a second spine 1142. First strut 1144 and a second strut 1146 form a first frame 1136 of ocular implant 1100 that extends between first spine 1140 and second spine 1142. In the exemplary embodiment of Figure 24, each strut undulates in a circumferential direction as it extends longitudinally between first spine 1140 and second spine 1142.

In the embodiment of Figure 24, the flexibility of body 1102 is at a maximum when body 1102 is bent by a moment acting about first axis 1156, and body 1102 has less flexibility when bent by a moment acting about an axis other than first axis 1156 (e.g., second axis 1158 and third axis 1160). Stated another way, the bending modulus of body 1102 is at a minimum when body 1102 is bent by a moment acting about first axis 1156, and body 1102 has a greater bending modulus when bent by a moment acting about an axis other than first axis 1156 (e.g., second axis 1158 and third axis 1160).

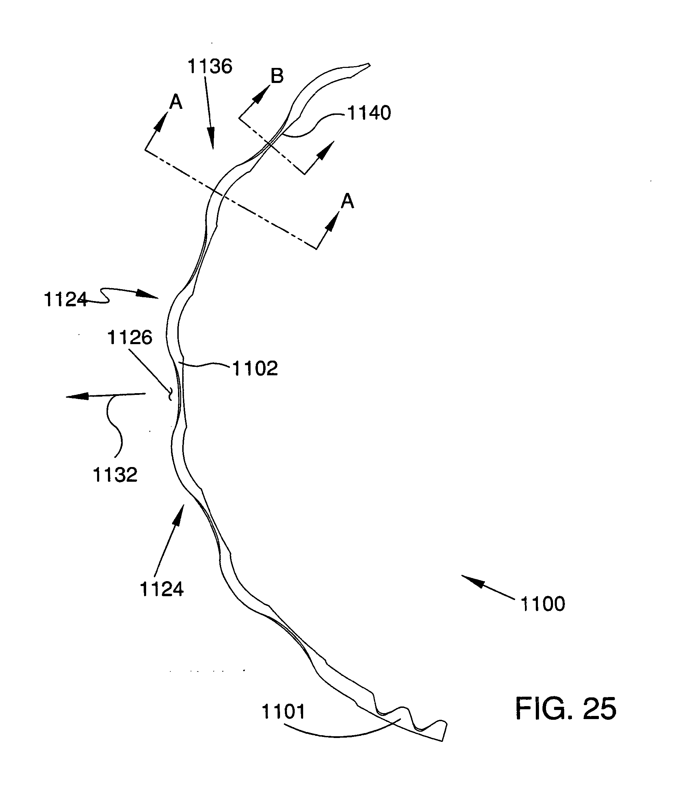

Figure 25 is a plan view showing ocular implant 1100 shown in the previous figure. In the embodiment of Figure 25, no external forces are acting on body 1102 of ocular implant 1100, and body 1102 is free to assume the generally curved resting shape depicted in Figure 25. Body 1102 defines a first opening 1124 that is disposed on an outer side 1130 of body 1102. A channel 1126 is defined by the inner surface of body 1102 and opens in a radially outward direction 1132 via first opening 1124. An inlet portion 1101 is formed at one end.

Section lines A-A and B-B are visible in Figure 25. Section line A-A intersects a first frame 1136 of ocular implant 1100. Section line B-B intersects a first spine 1140 of ocular implant 1100.

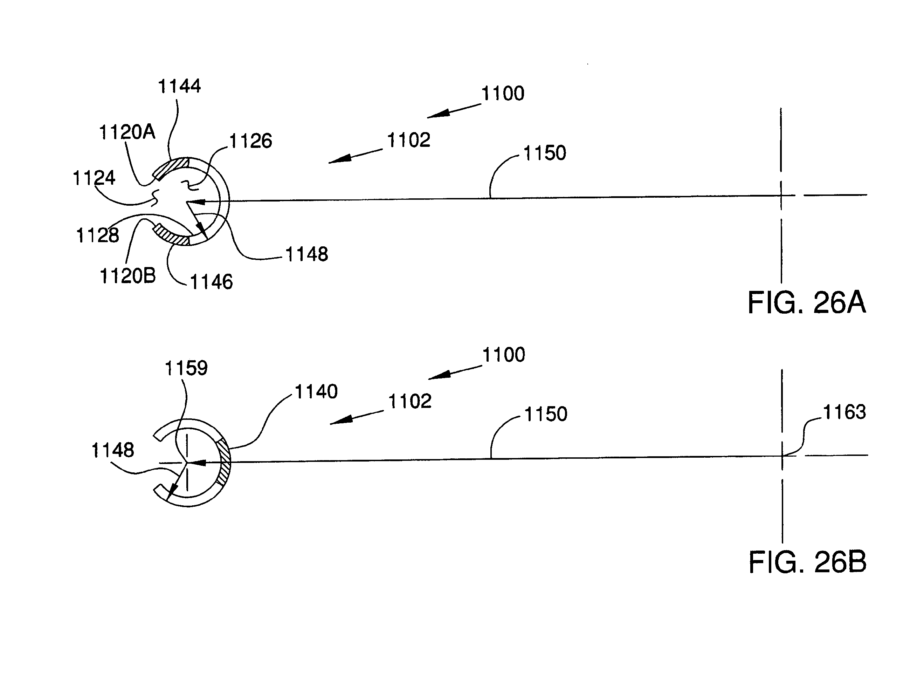

Figure 26A is a lateral cross-sectional view of ocular implant 1100 taken along section line A-A shown in the previous figure. Section line A-A intersects a first strut 1144 and a second strut 1146 of first frame 1136 at the point where the circumferential undulation of these struts is at its maximum. Body 1102 of ocular implant 1100 has a longitudinal radius 1150 and a lateral radius 1148. An inner surface 1128 of body 1102 defines a channel 1126. A first opening 1124 fluidly communicates with channel 1126.

In Figure 26A, first opening 1124 in body 1102 can be seen extending between first edge 1120A of first strut 1144 and a first edge 1120B of second strut 1146. With reference to Figure 26A, it will be appreciated that second strut 1146 has a shape that is a mirror image of the shape of first strut 1144.

Figure 26B is a lateral cross-sectional view of ocular implant 1100 taken along section line B-B shown in the previous figure. Section line B-B intersects first spine 1140 of ocular implant 1100. Body 1102 has a longitudinal radius 1150 and a lateral radius 1148. In the embodiment of Figure 26B, the center 1159 of lateral radius 1148 and the center 1163 of longitudinal radius 1150 are disposed on opposite sides of first spine 1140. The center 1159 of lateral radius 1148 is disposed on a first side of first spine 1140. The center 1163 of longitudinal radius 1150 is disposed on a second side of second spine 1142.

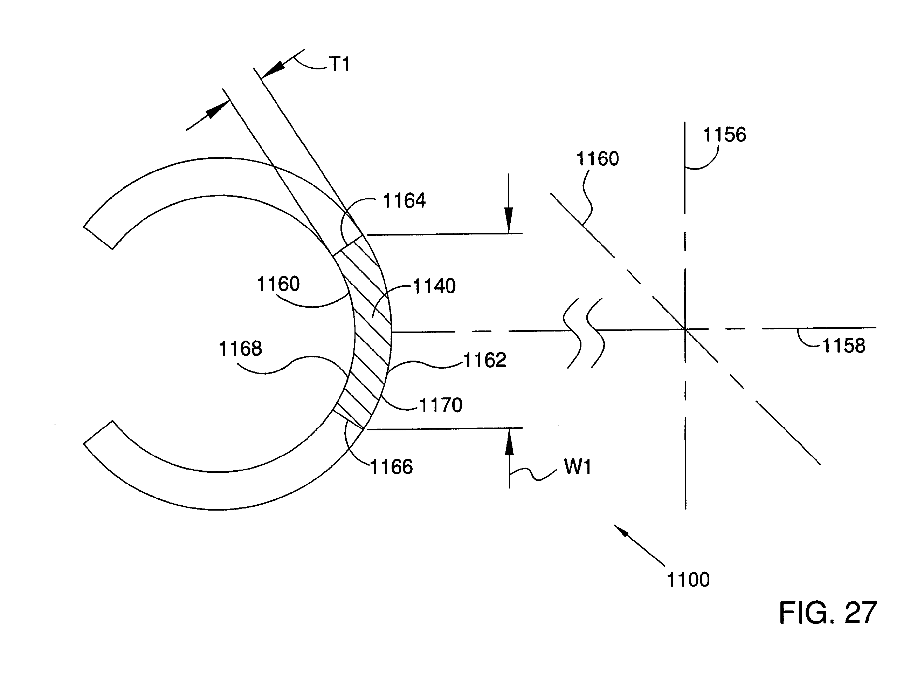

Figure 27 is an enlarged cross-sectional view of ocular implant 1100 taken along section line B-B of Figure 25. First spine 1140 includes a first major side 1160, a second major side 1162, a first minor side 1164, and second minor side 1166. With reference to Figure 27, it will be appreciated that first major side 1160 comprises a concave surface 1168. Second major side 1162 is opposite first major side 1160. In the embodiment of Figure 27, second major side 1162 comprises a convex surface 1170.

The geometry of the spine provides the ocular implant with flexibility characteristics that may aid in advancing the ocular implant into Schlemm's canal. In the embodiment of Figure 27, first spine 1140 has a thickness T1 extending between first major side 1160 and second major side 1162. Also in the embodiment of Figure 27, first spine 1140 has a width W1 extending between first minor side 1164 and second minor side 1166.

In some useful embodiments, the spine of an ocular implant in accordance with this detailed description has an aspect ratio of width W1 to thickness T1 greater than about 2. In some particularly useful embodiments, the spine of an ocular implant in accordance with this detailed description has an aspect ratio of width W1 to thickness T1 greater than about 4. In one useful embodiment, the ocular implant has a spine with an aspect ratio of width W1 to thickness T1 of about 5.2.

A first axis 1156, a second axis 1158 and a third axis 1160 are shown in Figure 27. Second axis 1158 is generally perpendicular to first axis 1156. Third axis 1160 is skewed relative to first axis 1156.

In the embodiment of Figure 27, the flexibility of first spine 1140 is at a maximum when first spine 1140 is bent by a moment acting about first axis 1156. First spine 1140 has a first flexibility when bent by a moment acting about first axis 156 and less flexibility when bent by a moment acting about an axis other than first axis 1156 (e.g., second axis 1158 and third axis 1160). For example, first spine 1140 has a second flexibility when bent by a moment acting about second axis 1158 shown in Figure 27. This second flexibility is less than the first flexibility that first spine 1140 has when bent by a moment acting about first axis 1156.

In the embodiment of Figure 27, the bending modulus of first spine 1140 is at a minimum when first spine 1140 is bent by a moment acting about first axis 1156. First spine 1140 has a first bending modulus when bent by a moment acting about first axis 1156 and a greater bending modulus when bent by a moment acting about an axis other than first axis 1156 (e.g., second axis 1158 and third axis 1160). For example, first spine 1140 has a second bending modulus when bent by a moment acting about second axis 1158 shown in Figure 27. This second bending modulus is greater than the first bending modulus that first spine 1140 has when bent by a moment acting about first axis 1156.

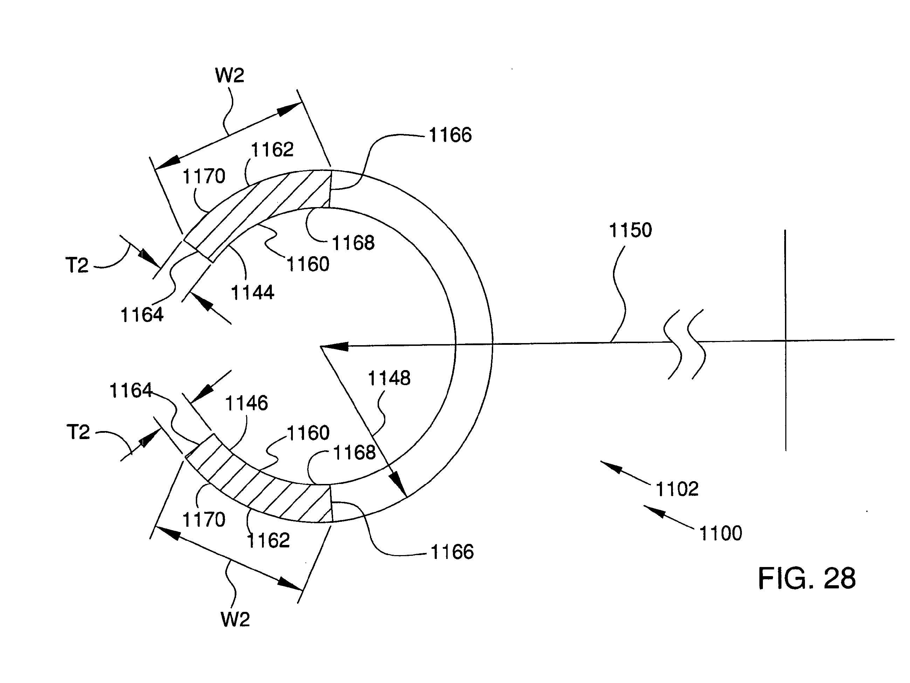

Figure 28 is an enlarged cross-sectional view of ocular implant 1100 taken along section line A-A of Figure 25. Section line A-A intersects first strut 1144 and second strut 1146 at the point where the circumferential undulation of these struts is at its maximum.

Each strut shown in Figure 28 includes a first major side 1160, a second major side 1162, a first minor side 1164, and second minor side 1166. With reference to Figure 28, it will be appreciated that each first major side 1160 comprises a concave surface 1168 and each second major side 1162 comprises a convex surface 1170.

In the embodiment of Figure 28, each strut has a thickness T2 extending between first major side 1160 and second major side 1162. Also in the embodiment of Figure 28, each strut has a width W2 extending between first minor side 1164 and second minor side 1166. In some useful embodiments, an ocular implant in accordance with this detailed description includes spines having a width W1 that is greater than the width W2 of the struts of the ocular implant.

In some useful embodiments, the struts of an ocular implant in accordance with this detailed description have an aspect ratio of width W2 to thickness T2 greater than about 2. In some particularly useful embodiments, the struts of an ocular implant in accordance with this detailed description have an aspect ratio of width W2 to thickness T2 greater than about 4. One exemplary ocular implant has struts with an aspect ratio of width W2 to thickness T2 of about 4.4.

Body 1102 of ocular implant 1100 has a longitudinal radius 1150 and a lateral radius 1148. In some useful embodiments, an ocular implant in accordance with this detailed description is sufficiently flexible to assume a shape matching the longitudinal curvature of Schlemm's canal when the ocular implant advanced into the eye. Also in some useful embodiments, a length of the ocular implant is selected so that the implant will extend across a pre-selected angular span when the implant is positioned in Schlemm's canal. Examples of pre-selected angular spans that may be suitable in some applications include 60°, 90°, 150° and 180°. The diameter of an ocular implant in accordance with this detailed description may be selected so that the ocular implant is dimensioned to lie within and support Schlemm's canal. In some useful embodiments, the diameter of the ocular implant ranges between about 0,127 mm (0.005 inches) and about 1,02 mm (0.04 inches) In some particularly useful embodiments, the diameter of the ocular implant ranges between about 0,127 mm (0.005 inches) and about 0,508 mm (0.02 inches)

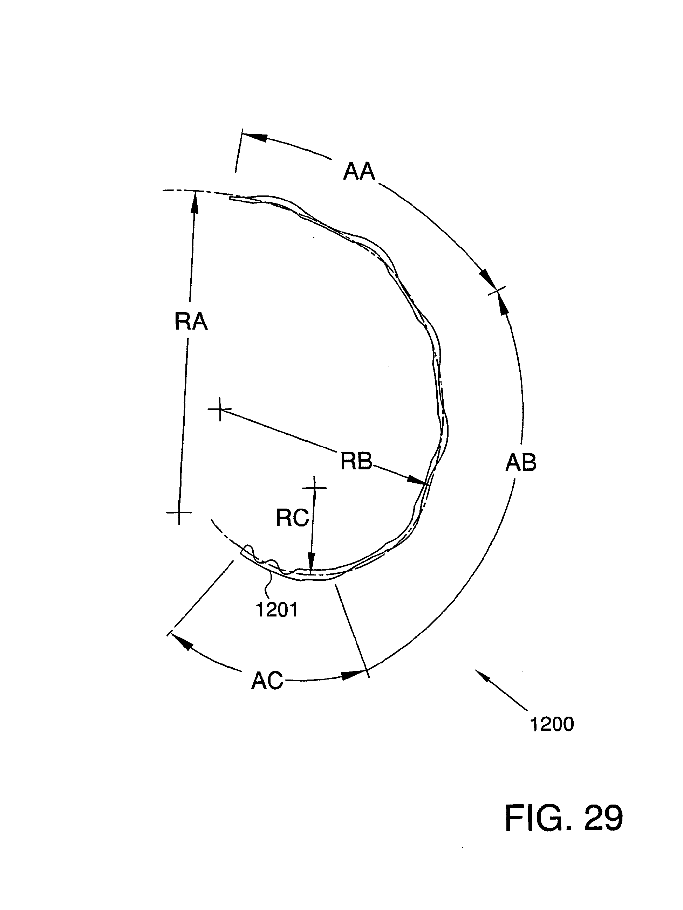

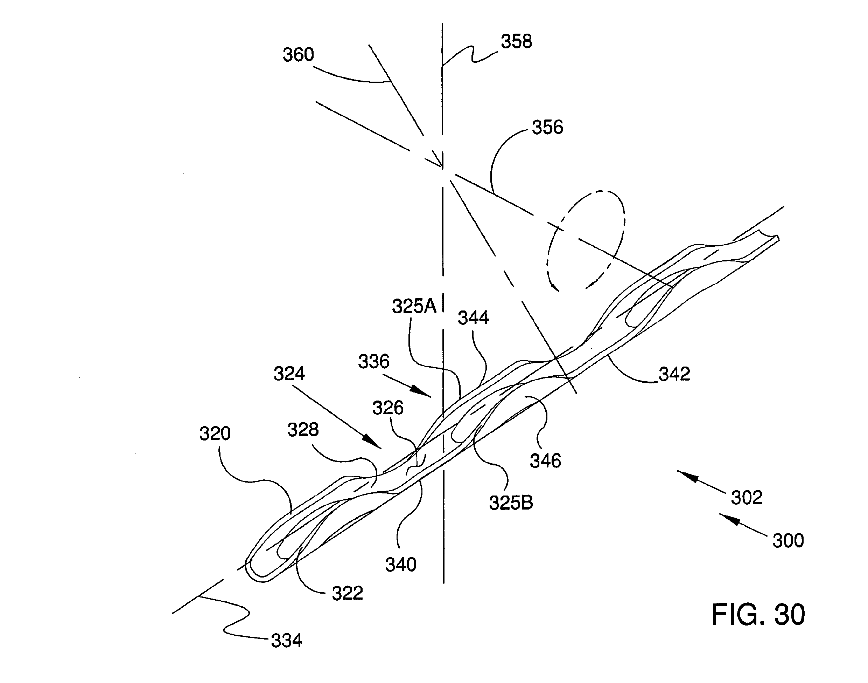

It is to be appreciated that an ocular implant in accordance with the present detailed description may be straight or curved. If the ocular implant is curved, it may have a substantially uniform longitudinal radius throughout its length, or the longitudinal radius of the ocular implant may vary along its length. Figure 25 shows one example of an ocular implant having a substantially uniform radius of curvature. Figure 29 shows one example of an ocular implant having a longitudinal radius of curvature that varies along the length of the ocular implant. An example of a substantially straight ocular implant is shown in Figure 30.

Figure 29 is a plan view showing an ocular implant 1200 having a longitudinal radius of curvature that varies along its length. In the embodiment of Figure 29, ocular implant 1200 has an at rest shape that is generally curved. This at rest shape can be established, for example, using a heat-setting process. The ocular implant shape shown in Figure 29 includes a distal radius RA, a proximal radius RC near inlet 1201, and an intermediate radius RB. In the embodiment of Figure 29, distal radius RA is larger than both intermediate radius RB and proximal radius RC. Also in the embodiment of Figure 29, intermediate radius RB is larger than proximal radius RC and smaller than distal radius RA..

In the embodiment of Figure 29, a distal portion of the ocular implant follows an arc extending across an angle AA. A proximal portion of the ocular implant follows an arc extending across an angle AC. An intermediate portion of the ocular implant is disposed between the proximal portion and the distal portion. The intermediate portion extends across an angle AB. In one useful embodiment, distal radius RA is about 8,13 mm (0.320 inches), intermediate radius RB is about 5,72 mm (0.225 inches), proximal radius RC is about 5,21 mm (0.205 inches), angle AA is about 55 degrees, angle AB is about degrees and angle AC is about 60 degrees. In another useful embodiment, distal radius RA is about 7,87 mm (0.310 inches), intermediate radius RB is about 5,46 mm (0.215 inches), proximal radius RC is about 2,67 mm (0.105 inches), angle AA is about 55 degrees, angle AB is about 79 degrees and angle AC is about 60 degrees.

Ocular implant 1200 may be used in conjunction with a method of treating the eye of a human patient for a disease and/or disorder (e.g., glaucoma). Some such methods may include the step of inserting a core member into a lumen defined by ocular implant 1200. The core member may comprise, for example, a wire or tube. The distal end of the ocular implant may be inserted into Schlemm's canal. The ocular implant and the core member may then be advanced into Schlemm's canal until the ocular implant has reached a desired position. The core member may then be withdrawn from the ocular implant. Further details of ocular implant delivery systems may be found in US Patent Appl. S.N. 11/943,289, filed November 20, 2007.

The flexibility and bending modulus features of the ocular implant of this invention may help ensure proper orientation of the implant within Schlemm's canal. Figure 20 shows the desired orientation of opening 1124 when the implant 1100 is disposed in Schlemm's canal. As shown, opening 1124 faces radially outward. The implant 1100 is therefore designed so that it is maximally flexible when bent along a plane defined by the longitudinal axis of implant 1100 as shown in Figure 20, and less flexible when bent in other planes, thereby enabling the curved shape of Schlemm's canal to help place the implant in this orientation automatically if the implant is initially placed in Schlemm's canal in a different orientation.

Figure 30 is a perspective view showing an ocular implant 300 in accordance with an additional embodiment in accordance with the present detailed description. With reference to Figure 30, it will be appreciated that ocular implant 300 has a resting (i.e., unstressed) shape that is generally straight. Ocular implant 300 extends along a longitudinal axis 334 that is generally straight. In some useful embodiments, ocular implant 300 is sufficiently flexible to assume a curved shape when advanced into Schlemm's canal of an eye.

Ocular implant 300 comprises a body 302. With reference to Figure 30, it will be appreciated that body 302 comprises a plurality of tissue supporting frames and a plurality of spines . As shown in Figure 30, these spines and frames are arranged in an alternating pattern in which one spine extends between each adjacent pair of frames . The frames of body 302 include a first frame 336 of ocular implant 300 is disposed between a first spine 340 and a second spine 342. In the embodiment of Figure 30, first frame 336 comprises a first strut 344 that extends between first spine 340 and second spine 342. A second strut 346 of first frame also extends between first spine 340 and second spine 342. Each strut undulates in a circumferential direction as it extends longitudinally between first spine 340 and second spine 342.

An inner surface 328 of body 302 defines a channel 326. Body 302 of ocular implant 300 includes a first edge 320 and a second edge 322 that define a first opening 324. Channel 326 of ocular implant 300 fluidly communicates with first opening 324. First strut 344 of first frame 336 comprises a first edge 325A. Second strut 346 has a first edge 325B. In Figure 30, first opening 324 in body 302 can be seen extending between first edge 325A of first strut 344 and a first edge 325B of second strut 346.

A first axis 356, a second axis 358 and a third axis 360 are shown in Figure 30. Second axis 358 is generally perpendicular to first axis 356. Third axis 360 is generally skewed relative to first axis 356. The flexibility of body 302 is at a maximum when body 302 is bent by a moment acting about first axis 356, and body 302 has less flexibility when bent by a moment acting about an axis other than first axis 356 (e.g., second axis 358 and third axis 360). Stated another way, in the embodiment of Figure 30, the bending modulus of body 302 is at a minimum when body 302 is bent by a moment acting about first axis 356, and body 302 has a greater bending modulus when bent by a moment acting about an axis other than first axis 356 (e.g., second axis 358 and third axis 360).

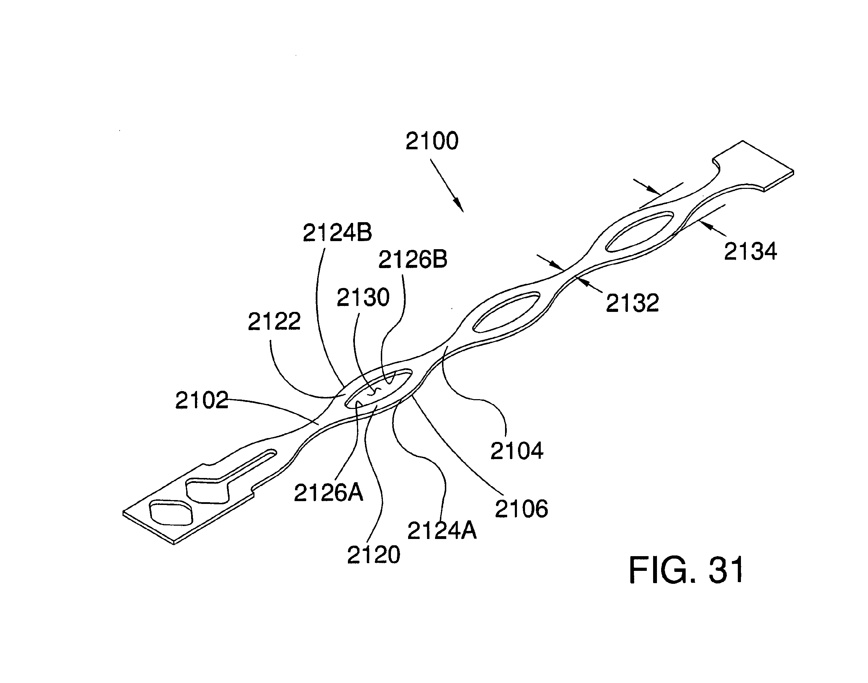

Figure 31 is an isometric view showing a body 2100 that may be used to form an ocular implant in accordance with one exemplary embodiment of the invention. Body 2100 comprises a first spine 2102, a second spine 2104, and a first frame 2106 disposed between first spine 2102 and second spine 2104. In the embodiment of Figure 31, first frame 2106 comprises a first strut 2120 and a second strut 2122. With reference to Figure 31, it will be appreciated that each strut extends between first spine 2102 and second spine 2104.

First strut 2120 of first frame 2106 comprises a first edge 2124A and a second edge 2126A. With reference to Figure 31, it will be appreciated that second strut 2122 has a shape that is a mirror image of the shape of first strut 2120. Second strut 2122 comprises a first edge 2124B and a second edge 2126B. Second edge 2126B of second strut 2122 and second edge 2126A of first strut 2120 define a second opening 2130. Second opening 2130 generally divides first frame 2106 into first strut 2120 and second strut 2122.

With continuing reference to Figure 31, it will be appreciated that body 2100 comprises a plurality of spines and a plurality of frames. In the embodiment of Figure 31, these spines and frames are arranged in an ABAB pattern. Each spine has a first lateral extent 2132 and each frame has a second lateral extent 2134, with second lateral extent 134 being greater than first lateral extent 132.

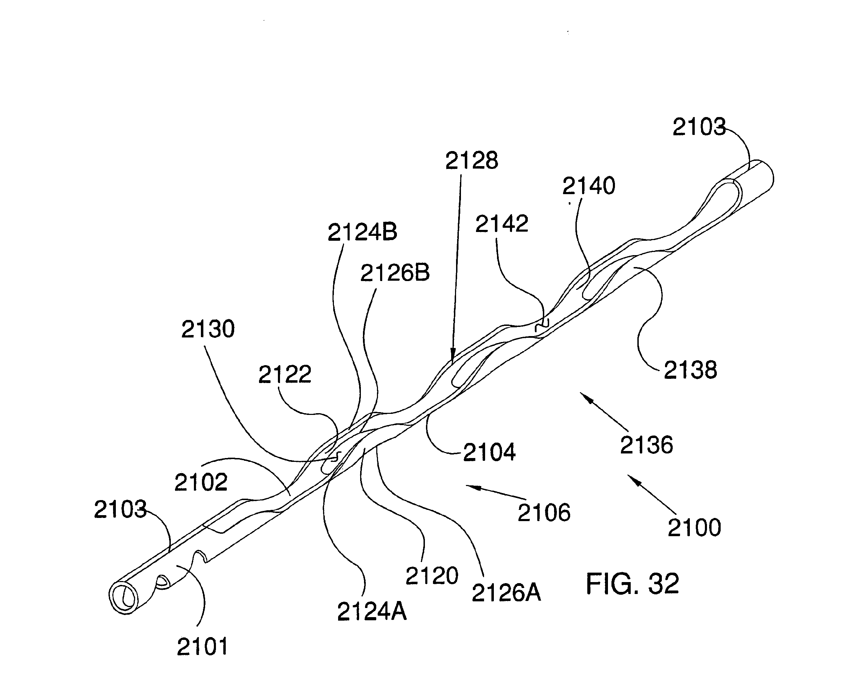

Figure 32 is an isometric view of body 2100 shown in the previous figure. In the embodiment of Figure 32, body 2100 is shaped to form an ocular implant 2136 having an outer surface 2138 defining a generally cylindrical volume. An inner surface 2140 of body 2100 defines an elongate channel 2142. Ocular implant 2136 may be inserted into Schlemm's canal of a human eye to facilitate the flow of aqueous humor out of the anterior chamber. This flow may include axial flow along Schlemm's canal, flow from the anterior chamber into Schlemm's canal, and flow leaving Schlemm's canal via outlets communicating with Schlemm's canal. When in place within the eye, ocular implant 2136 will support trabecular mesh tissue and Schlemm's canal tissue and will provide for improved communication between the anterior chamber and Schlemm's canal (via the trabecular meshwork) and between pockets or compartments along Schlemm's canal.

Elongate channel 2142 of ocular implant 2136 fluidly communicates with a first opening 2128 as well as inlet portion 2101. Various fabrication techniques may be used to fabricate ocular implant 2136. For example, ocular implant 2136 can be fabricated by providing a generally flat sheet of material and laser cutting the sheet of material to form body 2100 shown in Figure 31. The body 2100 may then be formed into a generally tubular shape as shown in Figure 32. Any adjoining edges (such as edges 2103) may be, optionally, welded. By way of a second example, ocular implant 2136 may be fabricated by providing a tube and laser cutting openings in the tube to form the shape shown in Figure 32.

As shown in Figure 32, ocular implant 2136 comprises a first spine 2102 and a second spine 2104. A first frame 2106 of ocular implant 2136 is disposed between first spine 2102 and second spine 2104. In the embodiment of Figure 32, first frame 2106 comprises a first strut 2120 that extends between first spine 2102 and second spine 2104. First frame 2106 also comprises a second strut 2122. Second strut 2122 also extends between first spine 2102 and second spine 2104.

First strut 2120 of first frame 2106 comprises a first edge 2124A and a second edge 2126A. Second strut 2122 has a shape that is a mirror image of the shape of first strut 2120. In Figure 32, first opening 2128 of ocular implant 2136 can be seen extending between first edge 2124A of first strut 2120 and a first edge 2124B of second strut 2122. A second edge 2126B of second strut 2122 and second edge 2126A of first strut 2120 define a second opening 2130. Second opening 2130 and additional openings (e.g., first opening 2128) defined by ocular implant 2136 allow aqueous humor to flow laterally across and/or laterally through ocular implant 2136.

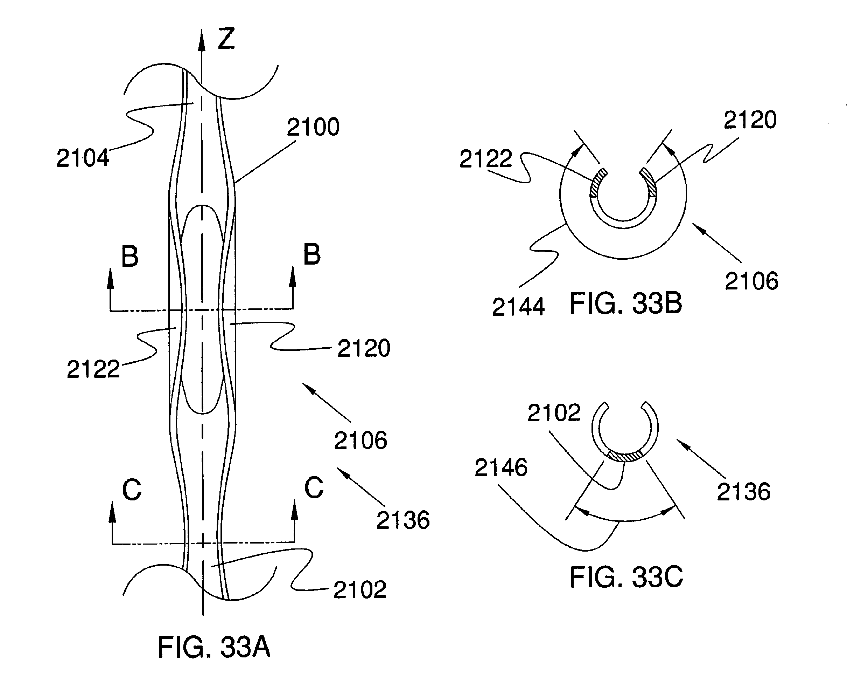

Figure 33A is a plan view showing a portion of ocular implant 2136 shown in the previous figure. Body 2100 of ocular implant 2136 comprises a first spine 2102, a second spine 2104, and a first frame 2106 disposed between first spine 2102 and second spine 2104. In the embodiment of Figure 33A, first frame 2106 comprises a first strut 2120 and a second strut 2122. As shown, each strut undulates in a circumferential direction while, at the same time, extending in the axial direction Z between first spine 2102 and second spine 2104.

Figure 33B is a lateral cross-sectional view of ocular implant 2136 taken along section line B-B. Section line B-B intersects first strut 2120 and second strut 2122 at the point where the circumferential undulation of these struts is at its maximum. First strut 2120 and second strut 2122 form first frame 2106. First frame 2106 has a first circumferential extent 2144 in the embodiment of Figure 33B.

Figure 33C is a lateral cross-sectional view of ocular implant 2136 taken along section line C-C. Section line C-C intersects first spine 2102 at the point where the width of first spine 2102 is at a minimum. At this point, first spine 2102 has a second circumferential extent 2146. Second circumferential extent 2146 of first spine 2102 is illustrated using dimension lines in Figure 33C. With reference to Figure 33C and Figure 33B, it will be appreciated that first circumferential extent 2144 of first frame 2106 is greater than second circumferential extent 2146 of first spine 2102.

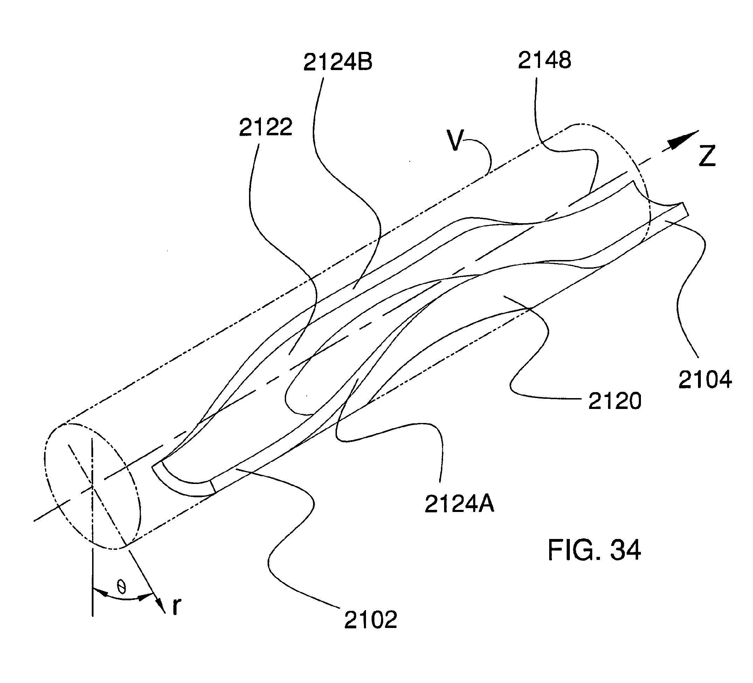

Figure 34 is an isometric view showing a portion of ocular implant 2136 shown in the previous figure. With reference to Figure 34, it will be appreciated that the outer surfaces of first spine 2102, second spine 2104, first strut 2120, and second strut 2122 define a portion of a generally cylindrical volume V. The shape of ocular implant 2136 may be described using the cylindrical coordinates shown in Figure 34. These cylindrical coordinates include a radius r, an angle θ and an axial dimension Z. Cylindrical coordinates may be conceptualized as an extension of two dimensional polar coordinates to include a longitudinal or axial dimension Z. The two dimensions of a typical polar coordinate system are radius r and angle θ. In the embodiment of Figure 34, dimension Z extends along a longitudinal axis 2148 of cylindrical volume V.

As shown in Figure 34, first strut 2120 extends in axial direction Z between first spine 2102 and second spine 2104. Second strut 2122 also extends between first spine 2102 and second spine 2104. In the embodiment of Figure 34, the radius r of the outer surface of each strut remains substantially constant. The angular dimension θ of a first edge 2124A of first strut varies as first strut 2120 extends in the axial direction Z between first spine 2102 and second spine 2104. Similarly, the angular dimension θ of a second edge 2126A of second strut varies as second strut 2122 extends in the axial direction Z between first spine 2102 and second spine 2104.

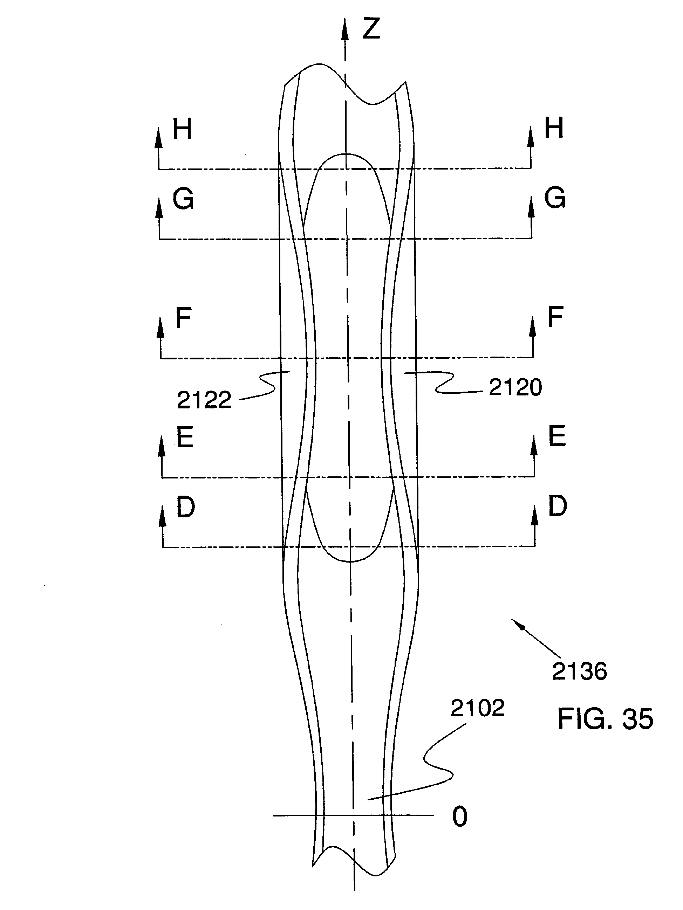

Figure 35 is an enlarged plan view showing a portion of ocular implant 2136 shown in the previous figure. In Figure 35, a number of section lines are shown crossing first strut 2120 and second strut 2122 of ocular implant 2136. In the embodiment of Figure 35, each strut undulates in a circumferential direction while, at the same time, extending in axial direction Z between first spine 2102 and second spine 2104. The circumferential undulation of first strut 2120 is illustrated in the next figure using lateral cross-sectional drawings labeled with cylindrical coordinates.

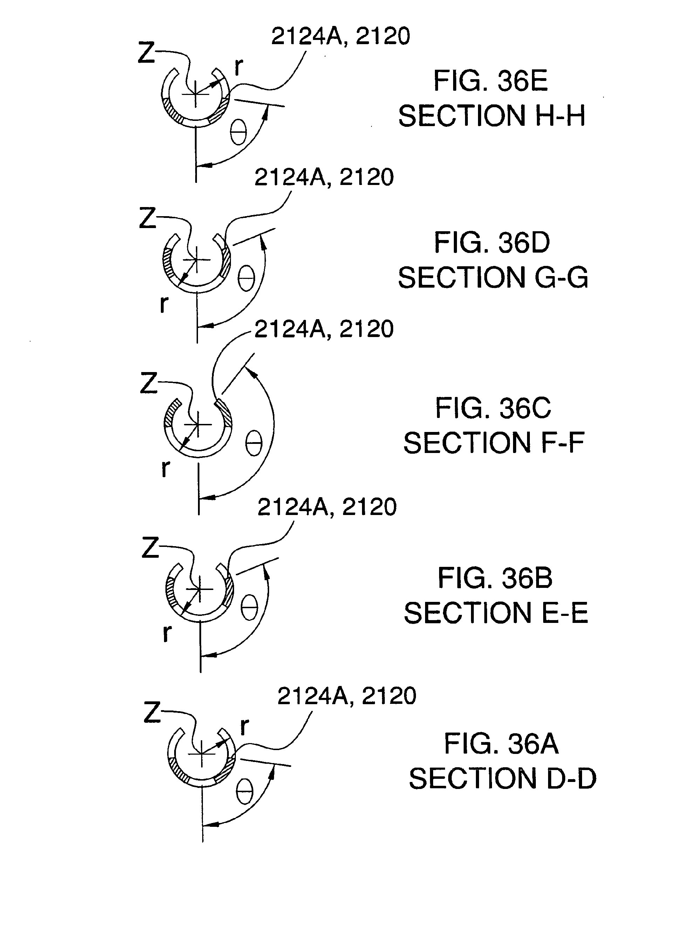

Figure 36A through 36E are lateral cross-sectional views of ocular implant 2136 shown in the previous figure. These cross-sectional views correspond to the section lines shown in the previous figure. With reference to these cross-sectional views, it will be appreciated that the angular dimension θ associated with first edge 2124A of first strut 2120 undulates as first strut 2120 extends in an axial direction Z between the first spine and the second spine. In the embodiment of Figure 36, the radius r of the outer surface of first strut 2120 remains substantially constant as first strut 2120 extends in axial direction Z between the first spine and the second spine.

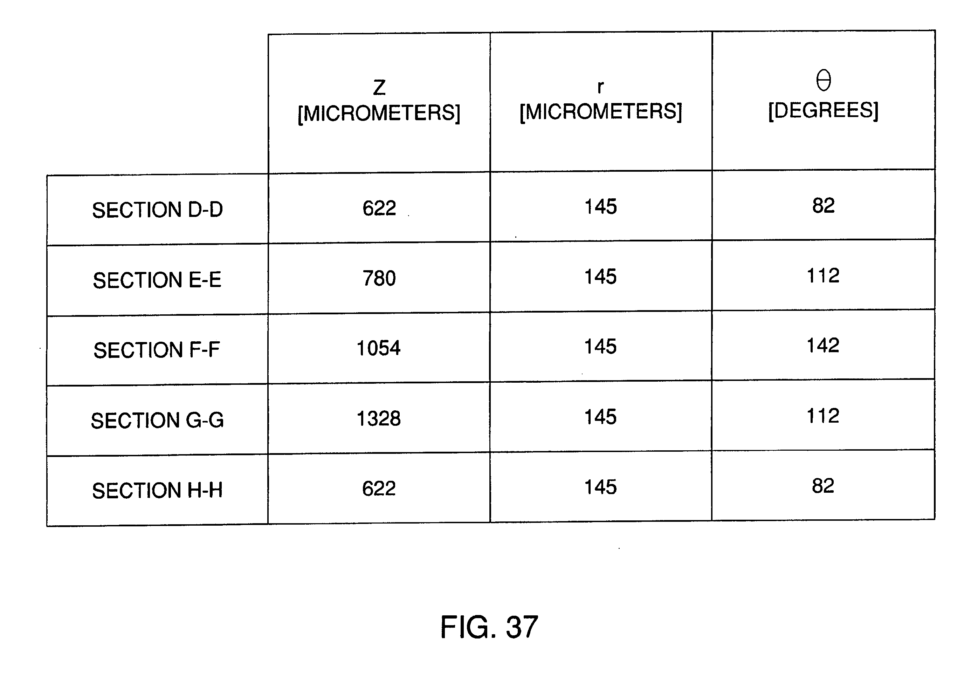

Figure 37 shows a plurality of cylindrical coordinate values corresponding with the cross-sectional views shown in the previous figure. With reference to the numerical values shown in Figure 37, it will be appreciated that the numerical value of angular dimension θ of first edge 2124 first increases, then decreases, as first strut 2120 extends in an axial direction Z between the first spine and the second spine. The numerical value r remains constant as first strut 2120 extends in axial direction Z between the first spine and the second spine.

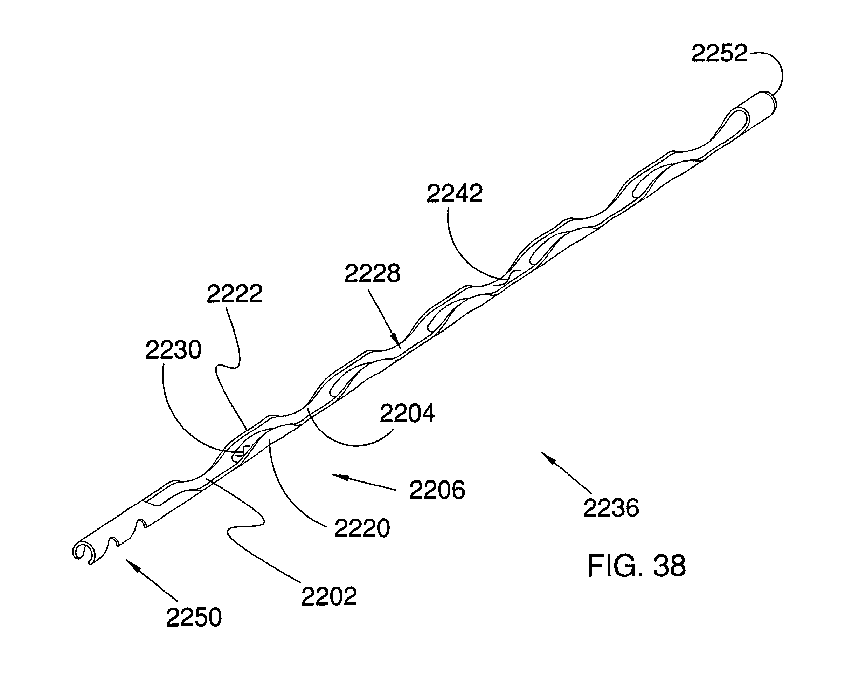

Figure 38 is an isometric view of an ocular implant 2236 in accordance with an additional exemplary embodiment of the invention. As shown in Figure 38, ocular implant 2236 comprises a first spine 2202 and a second spine 2204. A first frame 2206 of ocular implant 2236 is disposed between first spine 2202 and second spine 2204. In the embodiment of Figure 38, first frame 2206 comprises a first strut 2220 that extends between first spine 2202 and second spine 2204. First frame 2206 also comprises a second strut 2222. With reference to Figure 38, it will be appreciated that second strut 2222 also extends between first spine 2202 and second spine 2204.

Ocular implant 2236 of Figure 38 defines a channel 2242 that opens into a first opening 2228. In Figure 38, first opening 2228 of ocular implant 2236 can be seen extending between first strut 2220 and second strut 2222. First strut 2220 and second strut 2222 also define a second opening 2230. First opening 2228, second opening 2230, and the additional openings shown in Figure 38, allow aqueous humor to flow laterally across and/or laterally through ocular implant 2236.

In the embodiment of Figure 38, an inlet portion 2250 is formed near a proximal end of ocular implant 2236. Inlet portion 2250 may extend through the trabecular meshwork into the anterior chamber of the eye when a portion of the ocular implant lies in Schlemm's canal.

In the embodiment of Figure 38, a blunt tip 2252 is disposed at a distal end of ocular implant 2236. In some useful embodiments of ocular implant 2236, blunt tip 2252 has a generally rounded shape. In the embodiment shown in Figure 38, blunt tip 2252 has a generally hemispherical shape. The generally rounded shape of blunt tip 2252 may increase the likelihood that body 2200 will track Schlemm's canal as ocular implant 2236 is advanced into the canal during an implant procedure.

In Figure 38, ocular implant 2236 is pictured assuming a generally straight shape. Embodiments of ocular implant 2236 are possible which have a generally curved resting shape, as described above. Ocular implant 2236 may be fabricated, for example, by laser cutting a tube to create the shape shown in Figure 38. When this is the case, it may be desirable to rotate a straight tubular workpiece during the laser cutting process. After the laser cutting process, the ocular implant can be heat-set so that the ocular implant is biased to assume a selected shape (e.g., a generally curved shape).

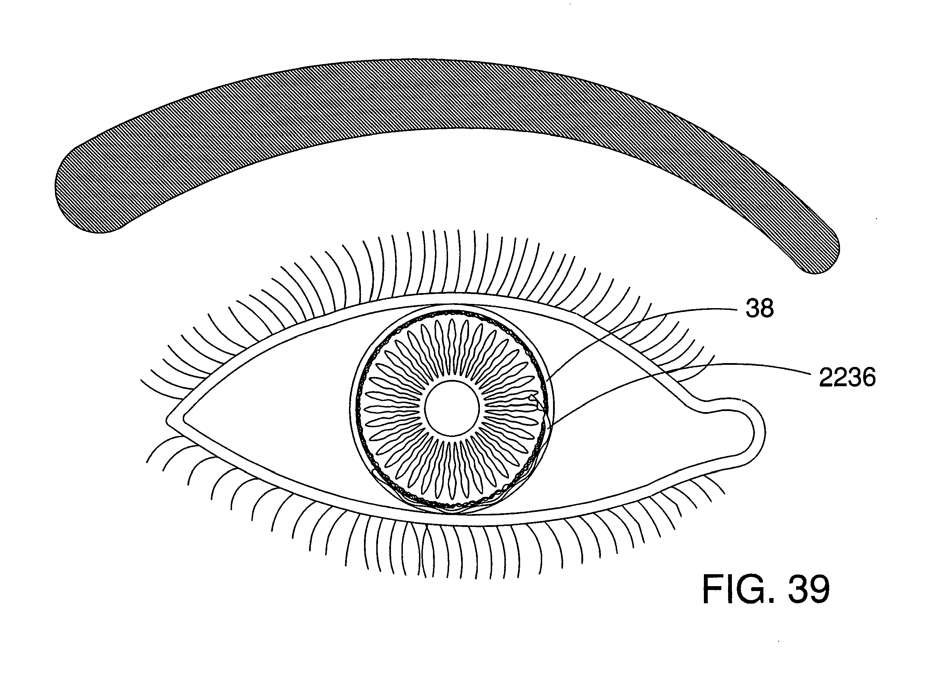

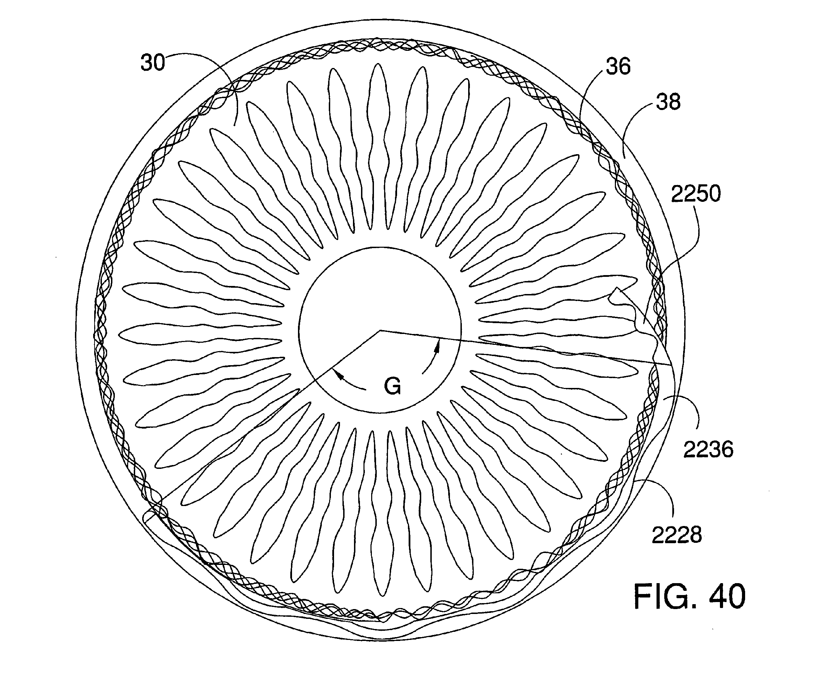

Figures 39 and 40 show an ocular implant 2236 according to this invention in place within a human eye. The eye of Figure 39 includes an anterior chamber 30 that is covered by a cornea. Schlemm's canal 38 can be seen encircling the iris of the eye. Ocular implant 2236 may be inserted into Schlemm's canal 38 to facilitate the flow of aqueous humor out of the anterior chamber. This flow may include axial flow along Schlemm's canal, flow from the anterior chamber into Schlemm's canal, and flow leaving Schlemm's canal via outlets communicating with Schlemm's canal. When in place within the eye, ocular implant 2236 will support trabecular mesh tissue 36 and Schlemm's canal tissue and will provide for improved communication between the anterior chamber 24 and Schlemm's canal 20 (via the trabecular meshwork 36) and between pockets or compartments along Schlemm's canal.

With reference to Figure 40, it will be appreciated that ocular implant 2236 extends through Schlemm's canal 38 across an angle G. Various implant sizes are possible, and different implant sizes may span a different angle G when placed in Schlemm's canal. Examples of angular spans that may be suitable in some applications include 60°, 90°, 150° and 180°.

In Figure 40, an inlet portion 2250 of ocular implant 2236 is shown extending through trabecular mesh 36. Aqueous humor may exit anterior chamber 30 and enter Schlemm's canal 38 by flowing through inlet portion 2250 of ocular implant 2236. Aqueous humor may also exit anterior chamber 30 and enter Schlemm's canal 38 by flowing through the trabecular mesh 36 of the eye. With reference to Figure 40, it will be appreciated that the spines of ocular implant 2236 support trabecular mesh 36.

As described above, aqueous humor exits Schlemm's canal by flowing through a number of outlets. After leaving Schlemm's canal, aqueous humor travels through a network passages and veins and is absorbed into the blood stream. Schlemm's canal typically has a non-circular cross-sectional shape whose diameter can vary along the canal's length and according to the angle at which the diameter is measured. In addition, there may be multiple partial pockets or partial compartments (not shown in these figures) formed along the length of Schlemm's canal. The shape and diameter of portions of Schlemm's canal and the existence and relative location of partial pockets or compartments may limit or prevent fluid flow from one point of Schlemm's canal to another. Hence, each outlet from Schlemm's canal may drain only a portion of Schlemm's canal. This condition may be improved by placing ocular implant 2236 in Schlemm's canal. Ocular implant 2236 shown in Figure 40 includes a plurality of struts, spines and openings. When in place within the eye, ocular implant 2236 will support trabecular mesh tissue and Schlemm's canal tissue and will provide for improved communication between the anterior chamber and Schlemm's canal and between pockets or compartments along Schlemm's canal.

In Figure 40, first opening 2228 of ocular implant 2236 is shown facing radially outward in Schlemm's canal 38. Aqueous humor can exit Schlemm's canal 38 by flowing through outlets that radiate away from and communicate with Schlemm's canal 38. After flowing through these outlets, this excess aqueous humor can enter the venous bloodstream be carried out of the eye by venous blood flow. The diameter of ocular implant 2236 can range from 0,127 mm (0.005 inches) to 1,02 mm (0.04 inches), preferably from 0,127 mm (0.005 inches) to 0,508 mm (0.02 inches), in order to lie within and support Schlemm's canal.

Various fabrication techniques may be used to fabricate the ocular implant. For example, the ocular implant can be fabricated by providing a generally flat sheet of material, cutting the sheet of material, and forming the material into a desired shape. By way of a second example, the ocular implant may be fabricated by providing a tube and laser cutting openings in the tube to form the ocular implant.

The ocular implants of this invention can be fabricated from various biocompatible materials possessing the necessary structural and mechanical attributes. Both metallic and non-metallic materials may be suitable. Examples of metallic materials include stainless steel, tantalum, gold, titanium, and nickel-titanium alloys known in the art as Nitinol. Nitinol is commercially available from Memry Technologies (Brookfield, Conn.), TiNi Alloy Company (San Leandro, Calif.), and Shape Memory Applications (Sunnyvale, Calif.). The ocular implants can be made, e.g., by laser cutting openings in a tube to form the desired open areas. Any desired bent shape made be formed, e.g., by heat setting.

The ocular implants may include one or more therapeutic agents. One or more therapeutic agents may, for example, be incorporated into a polymeric coating that is deposited onto the outer surfaces of the struts and spines of the ocular implant. The therapeutic agent may comprise, for example, an anti-glaucoma drug. Examples of anti-glaucoma drugs include prostaglandin analogs. Examples of prostaglandin analogs include latanprost.

While exemplary embodiments of the present invention have been shown and described, modifications may be made, and it is therefore intended in the appended claims to cover all such changes and modifications which fall within the scope of the invention.

## Claims

1. An ocular implant adapted to reside at least partially in a portion of Schlemm's canal of an eye, the implant comprising:
a body extending in a curved volume whose longitudinal axis forms an arc of a circle, and
a plurality of open areas and strut areas formed in the body, the open areas extending over more than 50% of a surface defining the curved volume, the strut areas surrounding the open areas,
the body having a diameter of between 0,127 mm (0.005 inches) and 1,02 mm (0.04 inche).

2. The implant of claim 1 wherein the open areas comprise a first longitudinal section extending along the curved volume.

3. The implant of claim 2 wherein the first longitudinal section includes the largest radius portion of the curved volume.

4. The implant of claim 2 wherein the open areas further comprise a plurality of openings formed on a second longitudinal section.

5. The implant of claim 4 further comprising spine sections disposed between the openings formed on the second longitudinal section.

6. The implant of claim 4 wherein the second longitudinal section is opposite the first longitudinal section.

7. The implant of claim 4 wherein the strut areas extend axially and circumferentially around the body from one side of the longitudinal section to the other side of the longitudinal section.

8. The implant of claim 4 wherein the open areas comprises open areas between the strut areas.

9. The implant of claim 1 wherein material coverage in circular cross-sections perpendicular to the longitudinal axis is less than 50% over greater than 90% of the implant.

10. The implant of claim 1 further comprising an inlet portion disposed at one end of the body in fluid communication with the body and extending inward from the circle arc.

11. The implant of claim 1 wherein the body has circumferential material coverage in cross-sections perpendicular to the longitudinal axis that is less than 360 degrees over an entire length of the body.

12. The implant of claim 5 wherein each strut in the strut areas follows a circumferentially undulating path while extending longitudinally between a first spine and a second spine.

13. The implant of claim 5 wherein each spine section has an arcuate shape in lateral cross section.

14. The implant of claim 1 wherein each strut in the strut areas has an arcuate shape in lateral cross section.

15. The implant of claim 1 further comprising a blunt tip disposed at one end.

## Patentansprüche

1. Augenimplantat, welches ausgestaltet ist, um sich zumindest teilweise in einem Abschnitt des Schlemm-Kanals eines Auges zu befinden, wobei das Implantat umfasst:
einen Körper, welcher sich in einem gebogenen Volumen erstreckt, dessen Längsachse einen Kreisbogen ausbildet, und
mehrere offene Bereiche und Strebenbereiche, welche in dem Körper ausgebildet sind, wobei sich die offenen Bereiche über mehr als 50 % einer Fläche, welche das gebogene Volumen definiert, erstrecken, wobei die Strebenbereiche die offenen Bereiche umgeben, wobei der Körper einen Durchmesser zwischen 0,127 mm und 1,02 mm aufweist.

2. Implantat nach Anspruch 1, **dadurch gekennzeichnet, dass** die offenen Bereiche einen ersten Längsabschnitt umfassen, welcher sich entlang des gebogenen Volumens erstreckt.

3. Implantat nach Anspruch 2, **dadurch gekennzeichnet, dass** der erste Längsabschnitt den Abschnitt mit dem größten Radius des gebogenen Volumens aufweist.

4. Implantat nach Anspruch 2, **dadurch gekennzeichnet, dass** die offenen Bereiche darüber hinaus mehrere Öffnungen umfassen, welche auf einem zweiten Längsabschnitt ausgebildet sind.

5. Implantat nach Anspruch 4, darüber hinaus Gratabschnitte umfassend, welche zwischen den Öffnungen, die auf dem zweiten Längsabschnitt ausgebildet sind, angeordnet sind.

6. Implantat nach Anspruch 4, **dadurch gekennzeichnet, dass** der zweite Längsabschnitt dem ersten Längsabschnitt gegenüberliegt.

7. Implantat nach Anspruch 4, **dadurch gekennzeichnet, dass** sich die Strebenbereiche axial und über den Umfang um den Körper von einer Seite des Längsabschnitts zu der anderen Seite des Längsabschnitts erstrecken.

8. Implantat nach Anspruch 4, **dadurch gekennzeichnet, dass** die offenen Bereiche offene Bereiche zwischen den Strebenbereichen umfassen.

9. Implantat nach Anspruch 1, **dadurch gekennzeichnet, dass** die Materialabdeckung über mehr als 90 % des Implantats in Kreisquerschnitten senkrecht zu der Längsachse geringer als 50 % ist.

10. Implantat nach Anspruch 1, darüber hinaus einen Einlassabschnitt umfassend, welcher an einem Ende des Körpers angeordnet ist, sich in Fluidkommunikation mit dem Körper befindet und sich von dem Kreisbogen nach innen erstreckt.

11. Implantat nach Anspruch 1, **dadurch gekennzeichnet, dass** der Körper in Umfangsrichtung eine Materialabdeckung in Querschnitten senkrecht zu der Längsachse aufweist, welche kleiner als 360 Grad über eine Gesamtlänge des Körpers ist.

12. Implantat nach Anspruch 5, **dadurch gekennzeichnet, dass** jede Strebe in den Strebenbereichen einer Wellenbahn in Umfangsrichtung folgt während sie sich in Längsrichtung zwischen einem ersten Grat und einem zweiten Grat erstreckt.

13. Implantat nach Anspruch 5, **dadurch gekennzeichnet, dass** jeder Gratabschnitt eine gebogene Form im seitlichen Querschnitt aufweist.

14. Implantat nach Anspruch 1, **dadurch gekennzeichnet, dass** jede Strebe in den Strebenbereichen eine gebogene Form im seitlichen Querschnitt aufweist.

15. Implantat nach Anspruch 1 darüber hinaus eine stumpfe Spitze umfassend, welche an einem Ende angeordnet ist.

## Revendications

1. Implant oculaire adapté pour résider au moins partiellement dans une partie du canal de Schlemm d'un oeil, l'implant comprenant :
un corps s'étendant dans un volume courbe dont l'axe longitudinal forme un arc de cercle, et
une pluralité de surfaces ouvertes et de branches formées dans le corps, les surfaces ouvertes s'étendant sur plus de 50% d'une surface définissant le volume courbe, les branches entourant les surfaces ouvertes,
le corps ayant un diamètre allant de 0,127 mm (0,005 pouce) et 1,02 mm (0,04 pouce).

2. Implant selon la revendication 1, dans lequel les surfaces ouvertes comprennent une première section longitudinale s'étendant le long du volume courbe.

3. Implant selon la revendication 2, dans lequel la première section longitudinale comprend la plus grande partie de rayon du volume courbe.

4. Implant selon la revendication 2, dans lequel les surfaces ouvertes comportent une pluralité d'ouvertures formées sur une seconde section longitudinale.

5. Implant selon la revendication 4, comprenant en outre des sections de pointe disposées entre les ouvertures formées sur la seconde section longitudinale.

6. Implant selon la revendication 4, dans lequel la seconde section longitudinale est située en face de la première section longitudinale.

7. Implant selon la revendication 4, dans lequel les branches s'étendent axialement et circonférentiellement autour du corps d'un côté de la section longitudinale à l'autre côté de la section longitudinale.

8. Implant selon la revendication 4, dans lequel les surfaces ouvertes comprennent des surfaces ouvertes entre les branches.

9. Implant selon la revendication 1, dans lequel la couverture de matériau dans des sections transversales circulaires perpendiculaires à l'axe longitudinal est inférieure à 50% sur plus de 90% de l'implant.

10. Implant selon la revendication 1, comprenant en outre une partie d'entrée placée au niveau d'une extrémité du corps en communication de fluide avec le corps et s'étendant vers l'intérieur depuis l'arc de cercle.

11. Implant selon la revendication 1, dans lequel le corps a une couverture de matériau circonférentielle dans des sections transversales perpendiculaires à l'axe longitudinal qui est inférieure à 360 degrés sur toute la longueur du corps.

12. Implant selon la revendication 5, dans lequel chacune des branches suit un chemin qui ondule de façon circonférentielle tout en s'étendant longitudinalement entre une première point et une seconde pointe.

13. Implant selon la revendication 5, dans lequel chaque section de pointe a une forme courbe en coupe transversale latérale.

14. Implant selon la revendication 1, dans lequel chaque branche des régions de branche a une forme courbe en coupe transversale latérale.

15. Implant selon la revendication 1, comprenant en outre une extrémité épointée située au niveau d'une extrémité.
